# EUROPEAN PATENT APPLICATION

(11) **EP 4 273 210 A1**
(43) Date of publication of application: **08.11.2023**
(21) Application number: 22171693.9
(22) Date of filing: 04.05.2022
(51) Int. Cl.: C11D 3/386

(54) **DETERGENT COMPOSITIONS CONTAINING ENZYMES**

(71) Applicant: The Procter & Gamble Company, Cincinnati, OH 45202 (US)
(72) Inventor: JONES, Catherine, Newcastle upon Tyne, NE1 7RU (GB); LANT, Neil Joseph, Newcastle upon Tyne, NE12 9BZ (GB); MOMIN, Nazarmohammad Gulamhussain, Newcastle upon Tyne, NE12 9BZ (GB); MORALES GARCIA, Ana L., Newcastle upon Tyne, NE12 9BZ (GB); VALENTINI, Alessandra, Newcastle upon Tyne, NE12 9BZ (GB); WILLATS, William G. T., Newcastle upon Tyne, NE15 0NS (GB); YAU, Hamish Chun Lam, Newcastle upon Tyne, NE12 9BZ (GB)
(74) Representative: P&G Patent Belgium UK

(57) **Abstract**

Detergent compositions particularly for laundry, comprising alginate lyase enzyme, and a booster enzyme selected from one or more of (i) Pel-ase enzyme; (ii) Psl-ase enzyme; (iii) endo-β-1,3(4)-glucanase enzyme; and (iv) mixtures thereof. Methods of treating fabrics by contacting the fabric with an aqueous wash liquor having the detergent composition therein. The compositions and methods are particularly for improving whiteness of a fabric, improved soil removal from a fabric, for malodour removal from a fabric, for anti-wrinkle benefits, collar and/or cuff cleaning, anti-redeposition benefits and/or for improved drying of a fabric.

## Description

### REFERENCE TO A SEQUENCE LISTING

This application contains a Sequence Listing in computer readable form. The computer readable form is incorporated herein by reference.

### FIELD OF THE INVENTION

The present invention relates to detergent compositions, particularly for laundry, comprising alginate lyase enzymes and booster enzymes. The invention also relates to methods of cleaning using the detergent compositions.

### BACKGROUND OF THE INVENTION

In cleaning applications, particularly for laundry, degradation of whiteness over time and soil or stain removal are continuing problems. There are many cleaning technologies aimed at mitigating such problems however, it is a constant challenge to provide improved efficacy and especially in an environmentally favourable manner. In automatic washing machines these problems are compounded by the increased use of low wash temperatures (e.g., cold water) and shorter washing cycles which reduce stain/soil removal efficacy of detergent compositions and exacerbate problems of redeposition of soil onto fabric surfaces during the washing process and whiteness loss over multiple washes. Thus, build-up of soil over time is a problem for both coloured and white fabrics but may be particularly noticeable on white or pale-coloured fabrics, for example around collars and cuffs where incomplete cleaning occurs. This can also be problematic as it may result in malodour. The compositions and methods of the invention are suitable for use in hand wash and automatic washing and laundry detergent compositions.

Thus, it is an object of the present invention to provide a detergent composition, particularly for laundry which can be used in a washing process, even at low temperatures and short wash times, which will counteract whiteness degradation and/or remove complex soils, for example to enable dingy soil removal, deep cleaning, removing yellowness, in particular cleaning collars and cuffs and/or whiteness improvement/counteracting whiteness loss, and that can even be useful at low temperatures and short wash times.

### SUMMARY OF THE INVENTION

The present invention provides a detergent composition, particularly for laundry, comprising (a) from 0.00005 to 5 wt% alginate lyase enzyme (active enzyme protein) and (b) 0.00005 wt% to 5 wt% enzyme (active enzyme protein) of a booster enzyme selected from one or more of (i) Pel-ase enzyme; (ii) Psl-ase enzyme; (iii) endo-β-1,3(4)-glucanase enzyme; and (iv) mixtures thereof. Preferably the composition also comprises from 1 to 60 wt% anionic surfactant.

The invention also provides a method of treating a fabric, the method comprising contacting a fabric with an aqueous wash liquor comprising an alginate lyase enzyme and one or more booster enzymes selected from (i) Pel-ase enzyme; (ii) Psl-ase enzyme, (iii) endo-β-1,3(4)-glucanase enzyme; and (iv) mixtures thereof, and optionally an anionic surfactant.

Preferably the aqueous wash liquor comprises anionic surfactant in an amount from 0.05g/l to 5g/l, preferably from 0.01g/l to 3g/l.

The fabric is typically contacted with the aqueous wash liquor at a temperature of 60°C or less, preferably at a temperature of 40°C or less or 35°C or less, most preferably at a temperature of 30°C or less or even 25°C or less; and (iii) optionally rinsing the surface. The compositions and methods herein are particularly useful for treating any fabric surface, synthetic or natural, including cotton, wool, silk, polyester, nylon, elastane or mixed fabric, such as polycotton.

The invention also relates to the use of a composition or method as described above for: improving whiteness of a fabric or counteracting whiteness loss; improved soil removal from a fabric; dingy soil removal; deep cleaning; removing or mitigating yellowness; cleaning collars and/or cuffs; malodour reduction or removal from a fabric; anti-wrinkle benefits on a fabric; improved drying of a fabric; soil anti-redeposition benefits. The composition and method described herein may also give rise to biofilm-disrupting effects.

### DETAILED DESCRIPTION OF THE INVENTION

### Definitions

Parent or Parent enzyme: The term "parent" or "parent enzyme" means an enzyme to which an alteration is made to produce the enzyme variants. The parent may be a naturally occurring (wild-type) polypeptide or a variant thereof. For example, the alginate lyase parent may be any of SEQ ID Nos: 1, 2, 3, 4, 5, 6 or 7 listed herein.

Sequence Identity: The relatedness between two amino acid sequences or between two nucleotide sequences is described by the parameter "sequence identity". For purposes of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows: (Identical Residues x 100)/(Length of Alignment - Total Number of Gaps in Alignment)

Alternatively, the parameters used may be gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows: (Identical Deoxyribonucleotides x 100)/(Length of Alignment - Total Number of Gaps in Alignment)

Variant: The term "variant" means a polypeptide having enzyme activity comprising an alteration/mutation, i.e., a substitution, insertion, and/or deletion, at one or more (e.g. several) positions relative to the parent. A substitution means a replacement of an amino acid occupying a position with a different amino acid; a deletion means removal of an amino acid occupying a position; and an insertion means adding 1-3 amino acids adjacent to and immediately following an amino acid occupying a position.

Wild-Type Enzyme: The term "wild-type" enzyme means an enzyme expressed by a naturally occurring microorganism, such as a bacterium, algae, yeast, or filamentous fungus found in nature.

### Detergent Composition

The detergent composition comprises (a) from 0.00005 to 5 wt% alginate lyase enzyme (active enzyme protein) and (b) 0.00005 wt% to 5 wt% enzyme (active enzyme protein) of a booster enzyme selected from one or more of (i) Pel-ase enzyme; (ii) Psl-ase enzyme; (iii) endo-β-1,3(4)-glucanase enzyme; and (iv) mixtures thereof. The detergent composition optionally comprises cleaning adjunct.

The detergent composition of the present invention is preferably a laundry detergent. The composition may be in the form of a composition for use in a main wash step or as pre-treatment or rinse-added cleaning composition for consumer or institutional use.

### Alginate Lyase Enzyme

The alginate lyase is preferably microbial in origin, preferably bacterial or algal (for example from brown seaweed *(Phaeophyceae),* such as *Ascophyllum, Laminara, Macrocystis*), most preferably bacterial. The alginate lyase enzyme may be obtained from *Aeromonas sp., Azotobacter sp., Bacillus sp., Flavobacterium sp., Klebsiella sp., Pseudomonas sp., Sphingomonas sp., Vibrio sp., Zobellia galactanivorans,* most preferably *Flavobacterium sp.*

Preferably the alginate lyase enzyme comprises alginate lyase selected from: alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 1; alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 2; alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 3; alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 4; alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 5; alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 6; alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 7; or mixtures thereof. Preferred alginate lyase enzyme comprises alginate lyase enzyme corresponding to the wild-type or preferably a variant of the wild-type of any one of SEQ ID NOs: 1, 2, 3, 4, 5, 6 or 7 listed herein, or mixtures thereof. SEQ ID NOs 6 and 7 and variants thereof, and mixtures thereof are particularly preferred.

When the alginate lyase enzyme is a variant of a parent amino acid sequence, the parent alginate lyase enzyme preferably has a sequence identity to the polypeptide of one or more of SEQ ID NOs: 1, 2, 3, 4, 5, 6 or 7 of at least 50 % or at least 60%, or at least 70% or at least 80%, such as at least 85%, at least 90%, e.g. at least 92%, at least 93%, at least 94%, at least 95%, at least 96%, at least 97%, at least 98%, at least 99, or 100%, which has alginate lyase enzyme activity. It may be preferred for the variant amino acid sequence to differ from the parent alginate lyase by no more than fifteen, or no more than ten amino acids, or no more than five, or four or three or two or one amino acid(s) from the polypeptide of one or more of SEQ ID NOs: 1, 2, 3, 4, 5, 6 or 7.

When the alginate lyase enzyme is a variant of a parent amino acid sequence, the parent may be obtained from a microorganism of any genus. For purposes of the present invention, the term "obtained from" as used herein in connection with a given source shall mean that the parent encoded by a polynucleotide is produced by the source or by a cell in which the polynucleotide from the source has been inserted. In one aspect, the parent is secreted extracellularly. Variants may be prepared using any mutagenesis procedure known in the art, such as site-directed mutagenesis, synthetic gene construction, semi-synthetic gene construction, random mutagenesis, shuffling, etc.

The alginate lyase may be from any polysaccharide lyase (PL) family including PL5, PL6, PL7, PL14, PL15, PL17, PL18, PL32, PL34, and PL36. Preferably the alginate lyase is from PL family 7.

Preferably the alginate lyase enzyme has activity towards poly(beta-D-mannuronate) (polyM activity) and activity towards poly(alpha-L-guluronate) (polyG activity). The alginate lyase enzyme may comprise a single alginate lyase enzyme to provide the polyM activity and polyG activity or may comprise two or more alginate lyase enzymes which in combination provide the polyM and polyG activity. Preferably, the alginate lyase comprises an enzyme having both polyM activity and polyG activity. Preferably the polyM activity as defined by Test Method 1 in the test section herein, is at least 0.1 absorbance units, preferably at least 0.15 absorbance units and most preferably at least 2 absorbance units. Preferably the polyG activity as defined by Test Method 1 in the test section herein, is at least 0.3 absorbance units, preferably at least 0.4 absorbance units, or at least 0.5 or even at least 0.6 absorbance units. The alginate lyase enzyme may be incorporated into the cleaning compositions and methods of the invention in the form of a substantially pure enzyme. Alternatively, in particular where the enzyme is a variant of a wild-type enzyme, the variant is not recovered, but rather a host cell expressing the enzyme is used as the source of the alginate lyase enzyme.

The alginate lyase enzyme may be in the form of a liquid or a dry composition. For instance, the composition may be in the form of a granulate or a microgranulate. The alginate lyase enzyme may be stabilized including by encapsulation, in accordance with methods known in the art.

The alginate lyase enzyme is present in the composition in an amount from 0.00005 to 5 wt% active enzyme protein, preferably from 0.0001 to 2 wt% active protein or from 0.0005 or from 0.001 to 1 wt% active protein, or to 0.5 wt% or to 0.1 wt% or to 0.05 wt% active enzyme protein.

Preferably the alginate lyase enzyme is present in the aqueous wash liquor in an amount of from O.Olppm to 1000 ppm of the enzyme, or from 0.05 or from 0.1ppm to 750 or 500ppm.

### Pel-ase Enzyme

The Pel-ase enzyme exhibits glycoside hydrolase activity towards Pel polysaccharide and typically or preferably belongs to the endo-alpha-1,4-polygalactosminidase class (EC 3.2.1.109) of enzymes, preferably having at least 60% or 65% or more preferably at least 70% or 75% or 80% or 85% or 90% or 95% up to 100% identity to one or more of SEQ ID NO: 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40.

Preferably the Pel-ase glycoside hydrolase is an isolated glycoside hydrolase.

Preferably the Pel-ase glycoside hydrolase enzyme is present in the cleaning composition in an amount from 0.001 to 1 wt% based on active protein in the composition, or from 0.005 to 0.5 wt% or from 0.01 to 0.25 wt% based on weight of the composition.

Preferably the Pel-ase glycoside hydrolase enzyme is present in the aqueous wash liquor in an amount of from O.Olppm to 1000 ppm enzyme, based on active protein or from 0.05 or from 0.1ppm to 750 or 500ppm.
The Pel-ase described herein may also give rise to biofilm-disrupting effects or soil anti-redeposition effects.

### Psl-ase Enzyme

The Psl-ase enzyme exhibits glycoside hydrolase activity towards Psl polysaccharide. The Psl-ase preferably has at least 60% or 65% or more preferably at least 70% or 75% or 80% or 85% or 90% or 95% up to 100% identity to one or more of SEQ ID NO: 41, 42, 43, 44, 45, 46, 47, 48, 49, 50, 51 or 52.

Preferably the Psl-ase glycoside hydrolase is an isolated glycoside hydrolase.

Preferably the Psl-ase glycoside hydrolase enzyme is present in the cleaning composition in 20 an amount from 0.001 to 1 wt%, or from 0.005 to 0.5 wt% or from 0.01 to 0.25 wt% based on active protein.

Preferably the Psl-ase glycoside hydrolase enzyme is present in a laundering aqueous liquor in an amount of from O.Olppm to 1000 ppm enzyme, based on active protein or from 0.05 or from 0.1ppm to 750 or 500ppm.

The composition comprising the Psl-ase glycoside hydrolase enzyme described herein may also give rise to/be useful for biofilm-disrupting effects or soil anti-redeposition effects.

### Endo-beta-1,3(4)-glucanase Enzyme

The endo-beta-1,3(4)-glucanase enzyme typically or preferably belongs to the class E.C. 3.2.1.6 and exhibits significant endo-beta-1,3-glucanase activity against polysaccharides with an endo-beta-1,3-glucan backbone (e.g. curdlan, pachyman, laminarin, scleroglucan, schizophyllan) and so-called 'mixed linkage' glucan polysaccharides that contain both beta-1,3- and beta-1,4 linkages such as those found in cereals such as oats and barley. Activity against both of these substrates can be confirmed using Test Method 2. The beta-1,3(4)-glucanase preferably has at least 60% or 65% or more preferably at least 70% or 75% or 80% or 85% or 90% or 95% up to 100% identity to one or more of SEQ ID NO: 53, 54, 55, 56, 57 or 58.

The booster enzyme comprises one or more of (i) Pel-ase enzyme; (ii) Psl-ase enzyme; (iii) endo-β-1,3(4)-glucanase enzyme; and (iv) mixtures thereof. The booster enzyme may comprise Pel-ase enzyme and Psl-ase enzyme, and optionally endo-β-1,3(4)-glucanase enzyme; Pel-ase enzyme and endo-β-1,3(4)- glucanase enzyme, and optionally Psl-ase; or endo-β-1,3(4)-glucanase enzyme and Psl-ase enzyme, and optionally Pel-ase enzyme.

The composition comprises optional cleaning adjunct. Typically, the cleaning adjunct will be present in the composition in an amount from 1 to 98.9 wt%, more typically from 5 to 90 wt% cleaning adjunct. Suitable cleaning adjuncts comprise: additional surfactants, builders, bleach ingredients, colorants, chelating agents, dye transfer agents, deposition aids, dispersants, additional enzymes, and enzyme stabilizers, catalytic materials, optical brighteners, photoactivators, fluorescers, fabric hueing agents (shading dyes), fabric conditioners, preformed peracids, polymeric dispersing agents, clay soil removal/anti-redeposition agents, filler salts, hydrotropes, brighteners, suds suppressors, structure elasticizing agents, fabric softeners, preservatives, anti-oxidants, anti-shrinkage agents, germicides, fungicides, anti-tarnish, anti-corrosion agents, alkalinity sources, solubilizing agents, carriers, processing aids, pigments, dyes, perfumes and pH control agents, encapsulates, polymers and mixtures thereof. For example, these may include: bleach ingredients such as bleach activators, bleach boosters such as imine bleach boosters, bleach catalysts, hydrogen peroxide, sources of hydrogen peroxide such as percarbonate and/or perborate, especially percarbonate coated with material such as carbonate and/or sulphate salt, silicate salt, borosilicate, and any mixture thereof, pre-formed peracid, including pre-formed peracid in encapsulated form, transition metal catalysts; suds suppressors or suppressor systems such as silicone based suds suppressors and/or fatty acid based suds suppressors; fabric-softeners such as clay, silicone and/or quaternary ammonium compounds; flocculants such as polyethylene oxide; dye transfer inhibitors such as polyvinylpyrrolidone, poly 4-vinylpyridine N-oxide and/or co-polymer of vinylpyrrolidone and vinylimidazole; fabric integrity components such as oligomers produced by the condensation of imidazole and epichlorhydrin; soil dispersants and soil anti-redeposition aids such as alkoxylated polyamines and ethoxylated ethyleneimine polymers; anti-redeposition components such as polyesters; carboxylate polymers such as maleic acid polymers or co-polymers of maleic and acrylic acid; perfumes such as perfume microcapsules, starch encapsulated accords, perfume spray-on; soap rings; aesthetic particles; aesthetic dyes; fillers such as sodium sulphate and/or citrus fibres, although it may be preferred for the composition to be substantially free of fillers; silicate salt such as sodium silicate, including 1.6R and 2.0R sodium silicate, or sodium metasilicate; co-polyesters of di-carboxylic acids and diols; cellulosic polymers such as methyl cellulose, carboxymethyl cellulose, hydroxyethoxycellulose, or other alkyl or alkylalkoxy cellulose; solvents such as 1,2 propanediol, monoethanolamine; diethylene glycol, ethanol, and any mixture thereof; hydrotropes such as sodium cumene sulphonate, sodium xylene sulphonate, sodium toluene sulphonate, and any mixtures; organic acids and salts thereof, such as citric acid/citrate salts; and any combination thereof. The composition may be such that the cleaning adjunct comprises one or more selected from the group consisting of (i) perfume microcapsule; (ii) fabric hueing agent; (iii) protease; (iv) amphiphilic cleaning polymer; (v) lipase, or (vi) mixtures thereof.

### Anionic Surfactant

The present inventors have found that the enzyme combination provides good soil breakdown, however the removal of the products of the breakdown of the substrates and soils containing them is improved by the presence of anionic surfactant. Therefore, the laundry detergent composition comprises from 1 to 60 wt% an anionic surfactant. Preferably the weight ratio of anionic surfactant to active alginate lyase enzyme protein is at least 500:1, preferably at least 1000:1 or at least 1500:1 or at least 2000:1, preferably being no greater than 500000:1, preferably no greater than 400000:1, or no greater than 200000:1 or up to 150000:1 or 100000:1, or 50000:1 or 10000:1. Preferably the weight ratio of anionic surfactant to active booster enzyme protein is at least 500:1, preferably at least 1000:1 or at least 1500:1 or at least 2000:1, preferably being no greater than 500000:1, preferably no greater than 400000:1, or no greater than 200000:1 or up to 150000:1 or 100000:1, or 50000:1 or 10000:1.

Preferred anionic surfactants are sulfonate and sulfate surfactants, preferably alkylbenzene sulphonates and/or (optionally alkoxylated) alkyl sulfates. Particularly preferred anionic surfactant comprises linear alkylbenzene sulfonates (LAS). Preferred alkyl sulfates comprise alkyl ether sulfates, especially C-9-15 alcohol ether sulfates, especially those having an average degree of ethoxylation from 0.5 to 7, preferably from 1 to 5, C8-C16 ester sulfates and C10-C14 ester sulfates, such as mono dodecyl ester sulfates. In a preferred composition the anionic surfactant comprises alkyl benzene sulphonate and optionally in addition, optionally ethoxylated alkyl sulfate, preferably having a degree of ethoxylation from 0 to 7, more preferably from 0.5 to 3. Isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ether sulfates (AES or AEOS or FES, also known as alcohol ethoxy sulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or salt of fatty acids (soap), and combinations thereof are also suitable anionic surfactants.

The anionic surfactant is preferably added to the detergent composition in the form of a salt. Preferred cations are alkali metal ions, such as sodium and potassium. However, the salt form of the anionic surfactant may be formed in situ by neutralization of the acid form of the surfactant with alkali such as sodium hydroxide or an amine, such as mono-, di-, or tri-ethanolamine. The composition preferably comprises from 1 to 60 weight % or from 1 to 50 wt% or 2 or 5 to 40 wt% of the composition, anionic surfactant. The surfactant preferably comprises a surfactant system comprising an anionic surfactant and in addition, one or more additional surfactants, which may be non-ionic including semi-polar and/or cationic and/or zwitterionic and/or ampholytic and/or amphoteric and/or semi-polar nonionic and/or mixtures thereof.

The invention also provides a cleaning composition comprising: from 0.00005 to 5 wt% (active enzyme protein) of an alginate lyase enzyme; 0.00005 to 5wt% booster enzyme (active enzyme protein) and a surfactant wherein the surfactant comprises an anionic and a nonionic surfactant, preferably having a weight ratio of anionic to nonionic of from 30:1 to 1:2, preferably from 20:1 to 2:3 or to 1:1.

Suitable nonionic surfactants include alcohol ethoxylates (AE), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or fatty acid glucamides, FAGA), as well as products available under the trade names SPAN and TWEEN, and combinations thereof. Alcohol ethoxylates are particularly preferred, preferably having a C9-18 or preferably a C12-15 alkyl chain and preferably having an average degree of ethoxylation from 3 to 9, more preferably from 3 to 7. Commercially available nonionic surfactants cleaning include Plurafac ^{™}, Lutensol^{™} and Pluronic^{™} from BASF, Dehypon^{™} series from Cognis and Genapol^{™} series from Clariant.

The detergent composition preferably comprises from 0.5wt% to about 40wt% of a non-ionic surfactant, preferably 1 to 30 wt% of the composition non-ionic surfactant.

The detergent composition preferably comprises one or more additional enzymes. Therefore, a preferred composition comprises (a) alginate lyase, (b) booster enzyme, and (c) one or more additional enzymes selected from the group consisting of aminopeptidase, amylase, carbohydrase, carboxypeptidase, catalase, cellulase, chitinase, cutinase, cyclodextrin glycosyltransferase, esterase, alpha-galactosidase, beta-galactosidase, glucoamylase, alpha-glucosidase, beta-glucosidase, haloperoxidase, hexosaminidase, invertase, laccase, lipase, mannanase, mannosidase, nuclease, oxidase, pectinolytic enzyme, peptidoglutaminase, peroxidase, phytase, polyphenoloxidase, proteolytic enzyme, transglutaminase, xylanase, xanthan lyase, xanthanase, endo-β-1,3-glucanase and mixtures thereof. Preferably the cleaning composition comprises additional enzyme selected from nuclease, such as DNase or RNase, mannanase, xanthan lyase, xanthanase, amylase and mixtures thereof.

Preferably the composition comprises additional enzymes selected from xanthan lyase, xanthanase, mannanase and mixtures thereof. Mannanase is particularly preferred.

The additional enzyme(s) may be produced, for example, by a microorganism belonging to the genus *Aspergillus, e.g., Aspergillus aculeatus*, *Aspergillus awamori*, *Aspergillus foetidus, Aspergillus fumigatus, Aspergillus japonicus, Aspergillus nidulans, Aspergillus niger,* or *Aspergillus oryzae; Fusarium, e.g., Fusarium bactridioides, Fusarium cerealis*, *Fusarium crookwellense*, *Fusarium culmorum, Fusarium graminearum, Fusarium graminum, Fusarium heterosporum, Fusarium negundi, Fusarium oxysporum, Fusarium reticulatum*, *Fusarium roseum, Fusarium sambucinum*, *Fusarium sarcochroum, Fusarium sulphureum, Fusarium toruloseum, Fusarium trichothecioides,* or *Fusarium venenatum*; *Humicola, e.g., Humicola insolens* or *Humicola lanuginosa*; or *Trichoderma, e.g., Trichoderma harzianum, Trichoderma koningii, Trichoderma longibrachiatum, Trichoderma reesei,* or *Trichoderma viride.*

Preferably the composition comprises a protease or mixture of more than one protease, a lipase or mixture of more than one lipase, a peroxidase or mixture of more than one peroxidase, one or more amylolytic enzymes, e.g., an alpha-amylase, glucoamylase, maltogenic amylase, and/or a cellulase or mixture thereof.

In general, the properties of the chosen enzyme(s) should be compatible with the selected detergent, (i.e., pH-optimum, compatibility with other enzymatic and non-enzymatic ingredients, etc.), and the enzyme(s) should be present in effective amounts. Preferably, the product of the invention comprises at least 0.01 mg, preferably from about 0.05 to about 10, more preferably from about 0.1 to about 6, especially from about 0.2 to about 5 mg of active further enzyme/ g of composition.

Proteases: The composition of the invention preferably comprises a protease. A mixture of two or more proteases can contribute to an enhanced cleaning across a broader temperature, cycle duration, and/or substrate range. Suitable proteases include metalloproteases and serine proteases, including neutral or alkaline microbial serine proteases, such as subtilisins (EC 3.4.21.62). Suitable proteases include those of animal, vegetable or microbial origin. In one aspect, such suitable protease may be of microbial origin. The suitable proteases include chemically or genetically modified mutants of the aforementioned suitable proteases. In one aspect, the suitable protease may be a serine protease, such as an alkaline microbial protease or/and a trypsin-type protease. Examples of suitable neutral or alkaline proteases include:
subtilisins (EC 3.4.21.62), especially those derived from *Bacillus,* such as *Bacillus sp., B. lentus, B. alkalophilus, B. subtilis, B. amyloliquefaciens, B. pumilus, B. gibsonii,* and *B. akibaii* described in WO2004067737, WO2015091989, WO2015091990, WO2015024739, WO2015143360, US 6,312,936 B1, US 5,679,630, US 4,760,025, DE102006022216A1, DE102006022224A1, WO2015089447, WO2015089441, WO2016066756, WO2016066757, WO2016069557, WO2016069563, WO2016069569 and WO2016174234. Specifically, mutations S9R, A15T, V66A, A188P, V199I, Q239R, N255D (savinase numbering system).
trypsin-type or chymotrypsin-type proteases, such as trypsin (e.g., of porcine or bovine origin), including the Fusarium protease described in WO 89/06270 and the chymotrypsin proteases derived from *Cellumonas* described in WO 05/052161 and WO 05/052146.
metalloproteases, especially those derived from *Bacillus amyloliquefaciens* decribed in WO07/044993A2; from *Bacillus, Brevibacillus, Thermoactinomyces, Geobacillus, Paenibacillus, Lysinibacillus* or *Streptomyces spp.* described in WO2014194032, WO2014194054 and WO2014194117; from *Kribella alluminosa* described in WO2015193488; and from *Streptomyces* and *Lysobacter* described in WO2016075078.
protease having at least 90% identity to the subtilase from *Bacillus sp.* TY145, NCIMB 40339, described in WO92/17577 (Novozymes A/S), including the variants of this *Bacillus sp* TY145 subtilase described in WO2015024739, and WO2016066757.

Especially preferred proteases for the cleaning composition of the invention are polypeptides having at least 90%, preferably at least 95%, more preferably at least 98%, even more preferably at least 99% and especially 100% identity with the wild-type enzyme from Bacillus lentus, comprising mutations in one or more, preferably two or more and more preferably three or more of the following positions, using the BPN' numbering system and amino acid abbreviations as illustrated in WO00/37627, which is incorporated herein by reference: S9R, A15T, V68A, N76D, N87S, S99D, S99SD, S99A, S101G, S101M, S103A, V104N/I, G118V, G118R, S128L, P129Q, S130A, Y167A, R170S, A194P, V205I, Q206L/D/E, Y209W, M222S, Q245R and/or M222S.

Most preferably the protease is selected from the group of proteases comprising the below mutations (BPN' numbering system) versus either the PB92 wild-type (SEQ ID NO:2 in WO 08/010925) or the subtilisin 309 wild-type (sequence as per PB92 backbone, except comprising a natural variation of N87S).
(i) G118V + S128L + P129Q + S130A
(ii) S101M + G118V + S128L + P129Q + S130A
(iii) N76D + N87R + G118R + S128L + P129Q + S130A + S188D + N248R
(iv) N76D + N87R + G118R + S128L + P129Q + S130A + S188D + V244R
(v) N76D + N87R + G118R + S128L + P129Q + S130A
(vi) V68A + N87S + S101G + V104N
(vii) S99AD
(viii) S9R+A15T+V68A+N218D+Q245R

Suitable commercially available protease enzymes include those sold under the trade names Alcalase^{®}, Savinase^{®}, Primase^{®}, Durazym^{®}, Polarzyme^{®}, Kannase^{®}, Liquanase^{®}, Liquanase Ultra^{®}, Savinase Ultra^{®}, Ovozyme^{®}, Neutrase^{®}, Everlase^{®}, Coronase^{®}, Blaze^{®}, Blaze Ultra^{®} and Esperase^{®} by Novozymes A/S (Denmark); those sold under the tradename Maxatase^{®}, Maxacal^{®}, Maxapem^{®}, Properase^{®}, Purafect^{®}, Purafect Prime^{®}, Purafect Ox^{®}, FN3^{®}, FN4^{®}, Excellase^{®}, Ultimase^{®} and Purafect OXP^{®} by Dupont; those sold under the tradename Opticlean^{®} and Optimase^{®} by Solvay Enzymes; and those available from Henkel/Kemira, namely BLAP (sequence shown in Figure29 of US 5,352,604 with the following mutations S99D + S101 R + S103A + V104I + G159S, hereinafter referred to as BLAP), BLAP R (BLAP with S3T + V4I + V199M + V205I + L217D), BLAP X (BLAP with S3T + V4I + V205I) and BLAP F49 (BLAP with S3T + V4I + A194P + V199M + V205I + L217D); and KAP (Bacillus alkalophilus subtilisin with mutations A230V + S256G + S259N) from Kao.

Especially preferred for use herein are commercial proteases selected from the group consisting of Properase^{®}, Blaze^{®}, Ultimase^{®}, Everlase^{®}, Savinase^{®}, Excellase^{®}, Blaze Ultra^{®}, BLAP and BLAP variants.

Preferred levels of protease in the product of the invention include from about 0.05 to about 10, more preferably from about 0.5 to about 7 and especially from about 1 to about 6 mg of active protease/g of composition.

Lipases: The composition preferably comprises a lipase. The presence of oils and/or grease can further increase the resiliency of stains comprising mannans and other polysaccharides. As such, the presence of lipase in the enzyme package can further improve the removal of such stains. Suitable lipases include those of bacterial or fungal or synthetic origin. Chemically modified or protein engineered mutants are included. Examples of useful lipases include lipases from *Humicola* (synonym *Thermomyces*), e.g., from *H*. *lanuginosa* (*T. lanuginosus*) or from *H. insolens,* a *Pseudomonas lipase,* e.g., from *P. alcaligenes* or *P. pseudoalcaligenes*, *P. cepacia P. stutzeri, P. fluorescens, Pseudomonas* sp. strain SD 705, *P. wisconsinensis* , a *Bacillus* lipase, e.g., from *B. subtilis* (Dartois et al. (1993), Biochemica et Biophysica Acta, 1131, 253-360), *B. stearothermophilus* or *B. pumilus .*

The lipase may be a "first cycle lipase" such as those described in U.S. Patent 6,939,702 B1 and US PA 2009/0217464. In one aspect, the lipase is a first-wash lipase, preferably a variant of the wild-type lipase from *Thermomyces lanuginosus* comprising T231R and N233R mutations. The wild-type sequence is the 269 amino acids (amino acids 23 - 291) of the Swissprot accession number Swiss-Prot O59952 (derived from *Thermomyces lanuginosus (Humicola lanuginosa)).* Preferred lipases include those sold under the tradenames Lipex^{®}, Lipolex^{®} and Lipoclean^{®}.

Other suitable lipases include: Liprl 139, e.g. as described in WO2013/171241; TfuLip2, e.g. as described in WO2011/084412 and WO2013/033318; Pseudomonas stutzeri lipase, e.g. as described in WO2018228880; *Microbulbifer thermotolerans* lipase, e.g. as described in WO2018228881; *Sulfobacillus acidocaldarius* lipase, e.g. as described in EP3299457; LIP062 lipase e.g. as described in WO2018209026; PinLip lipase e.g. as described in WO2017036901 and Absidia sp. lipase e.g. as described in WO2017005798.

A suitable lipase is a variant of SEQ ID NO:5 comprising:
(a) substitution T231R
   and
(b) substitution N233R or N233C
   and
(c) at least three further substitutions selected from E1C, D27R, N33Q, G38A, F51V, G91Q, D96E, K98L, K98I, D111A, G163K, H198S, E210Q, Y220F, D254S, I255A, and P256T;
where the positions correspond to the positions of SEQ ID NO:5 and wherein the lipase variant has at least 90% but less than 100% sequence identity to the polypeptide having the amino acid sequence of SEQ ID NO: 5 and wherein the variant has lipase activity.

One preferred lipase is a variant of SEQ ID NO: 5 comprising the following substitutions: T231R, N233R, D27R, G38A, D96E, D111A, G163K, D254S and P256T

One preferred lipase is a variant of SEQ ID NO: 5 comprising the following substitutions: T231R, N233R, N33Q, G91Q, E210Q, I255A.

Suitable lipases are commercially available from Novozymes, for example as Lipex Evity 100L, Lipex Evity 200L (both liquid raw materials) and Lipex Evity 105T (a granulate). These lipases have different structures to the products Lipex 100L, Lipex 100T and Lipex Evity 100T which are outside the scope of the invention.

Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium,* e.g., the fungal cellulases produced from *Humicola insolens, Myceliophthora thermophila* and *Fusarium oxysporum.* disclosed in US 4,435,307 , US 5,648,263 , US 5,691,178, US 5,776,757 and US 5,691,178 .

In one aspect, preferred enzymes include microbial-derived endoglucanases exhibiting endo-beta-1,4-glucanase activity (E.C. 3.2.1.4), preferably selected from the group comprising:
(a) a bacterial polypeptide endogenous to a member of the genus Bacillus which has a sequence of at least 90%, 94%, 97% and even 99% identity to the amino acid sequence SEQ ID NO:2 in US 7,141,403B2, preferred substitutions compriseone or more positions corresponding to positions 292, 274, 266, 265, 255, 246, 237, 224 and 221 of the mature polypeptide of SEQ ID NO: 2, and t he variant has cellulase activity.;
(b) a glycosyl hydrolase having enzymatic activity towards both xyloglucan and amorphous cellulose substrates, wherein the glycosyl hydrolase is selected from GH families 5, 7, 12, 16, 44 or 74;
(c) a glycosyl hydrolase having a sequence of at least 90%, 94%, 97% and even 99% identity to the amino acid sequence SEQ ID NO:3 in WO09/148983;
(d) Variants exhibiting at least 70% identity with SEQ ID NO: 5 in WO2017106676. Preferred substitutions comprise one or more positions corresponding to positions 4, 20, 23, 29, 32, 36, 44, 51, 77, 80, 87, 90, 97, 98, 99, 102, 112, 116, 135, 136, 142, 153, 154, 157, 161, 163, 192, 194, 204, 208, 210, 212, 216, 217, 221, 222, 225, 227, and 232;
(e) and mixtures thereof.

Suitable endoglucanases are sold under the tradenames Celluclean^{®} and Whitezyme^{®} (Novozymes A/S, Bagsvaerd, Denmark). Examples include Celluclean^{®} 5000L, Celluclean^{®} Classic 400L, Celluclean^{®} Classic 700T, Celluclean^{®} 4500T, Whitezyme^{®} 1.5T, Whitezyme^{®} 2.0L.

Other commercially available cellulases include Celluzyme^{®}, Carezyme^{®}, Carezyme^{®} Premium (Novozymes A/S), Clazinase^{®}, Puradax HA^{®}, Revitalenz^{®} 1000, Revitalenz^{®} 2000 (Genencor International Inc.), KAC-500(B)^{®} (Kao Corporation), Biotouch^{®} FCL, Biotouch^{®} DCL, Biotouch^{®} DCC, Biotouch^{®} NCD, Biotouch^{®} FCC, Biotouch^{®} FLX1 (AB Enzymes)

Suitable glucanases include endo-β-1,3-glucanases, preferably from E.C. class 3.2.1.39, preferably obtained from *Paenibacillus sp, Zobellia galactanivorans, Thermotoga petrophila* or *Trichoderma sp* micro-organism, preferably *Paenibacillus sp* or *Zobellia galactanivorans,* most preferably *Paenibacillus sp.*

Amylases: Preferably the composition of the invention comprises an amylase. Suitable alpha-amylases include those of bacterial or fungal origin. Chemically or genetically modified mutants (variants) are included. A preferred alkaline alpha-amylase is derived from a strain of Bacillus, such as *Bacillus licheniformis, Bacillus amyloliquefaciens, Bacillus stearothermophilus, Bacillus subtilis,* or other *Bacillus sp.,* such as Bacillus sp. NCBI 12289, NCBI 12512, NCBI 12513, DSM 9375 (USP 7,153,818) DSM 12368, DSMZ no. 12649, KSM AP1378 (WO 97/00324), KSM K36 or KSM K38 (EP 1,022,334). Preferred amylases include:
(a) variants described in USP 5,856,164 and WO99/23211, WO 96/23873, WO00/60060, WO06/002643 and WO2017/192657, especially the variants with one or more substitutions in the following positions versus the AA560 enzyme listed as SEQ ID No. 12 in WO 06/002643: 26, 30, 33, 82, 37, 106, 118, 128, 133, 149, 150, 160, 178, 182, 186, 193, 202, 214, 231, 246, 256, 257, 258, 269, 270, 272, 283, 295, 296, 298, 299, 303, 304, 305, 311, 314, 315, 318, 319, 339, 345, 361, 378, 383, 419, 421, 437, 441, 444, 445, 446, 447, 450, 461, 471, 482, 484, preferably that also contain the deletions of D183^{∗} and G184^{∗}.
(b) variants exhibiting at least 85%, preferably 90% identity with SEQ ID No. 4 in WO06/002643, the wild-type enzyme from Bacillus SP722, especially variants with deletions in the 183 and 184 positions and variants described in WO 00/60060, WO2011/100410 and WO2013/003659, particularly those with one or more substitutions at the following positions versus SEQ ID No. 4 in WO06/002643 which are incorporated herein by reference: 51, 52, 54, 109, 304, 140, 189, 134, 195, 206, 243, 260, 262, 284, 347, 439, 469, 476 and 477.
(c) variants exhibiting at least 90% identity with the wild-type enzyme from Bacillus sp.707 (SEQ ID NO:7 in US 6,093,562), especially those comprising one or more of the following mutations M202, M208, S255, R172, and/or M261. Preferably said amylase comprises one or more of M202L, M202V, M202S, M202T, M202I, M202Q, M202W, S255N and/or R172Q. Particularly preferred are those comprising the M202L or M202T mutations. Additional relevant mutations/deletions based on SP707 backbone include W48, A51, V103, V104, A113, R118, N125, V131, T132, E134, T136, E138, R142, S154, V165, R182, G182, H183, E190, D192, T193, 1206, M208, D209, E212, V213, V214, N214, L217, R218, N219, V222, T225, T227, G229, 1235, K242, Y243, S244, F245, T246, 1250, S255, A256, H286, V291, T316, V317, V318, N417, T418, A419, H420, P421, 1428, M429, F440, R443, N444, K445, Q448, S451, A465, N470, S472.
(d) variants described in WO 09/149130, preferably those exhibiting at least 90% identity with SEQ ID NO: 1 or SEQ ID NO:2 in WO 09/149130, the wild-type enzyme from *Geobacillus Stearophermophilus* or a truncated version thereof.
(e) variants described in WO10/115021, especially those exhibiting at least 75%, or at least 85% or at least 90% or at least 95% with SEQ ID NO:2 in WO10/115021, the alpha-amylase derived from *Bacillus sp.* TS-23.
(f) variants exhibiting at least 89% identity with SEQ ID NO:1 in WO2016091688, especially those comprising deletions at positions H183+G184 and additionally one or more mutations at positions 405, 421, 422 and/or 428.
(g) variants described in WO2014099523, especially those exhibiting at least 60% amino acid sequence identity with the "PcuAmyl α-amylase" from *Paenibacillus curdlanolyticus* YK9 (SEQ ID NO:3 in WO2014099523).
(h) variants described in WO2014099523, especially those exhibiting at least 60% amino acid sequence identity with the "CspAmy2 amylase" from *Cytophaga sp.* (SEQ ID NO:1 & 6 in WO2014164777. Especially those comprising one of more of the following deletions and/or mutations based on SEQ ID NO:1 in WO2014164777: R178^{∗}, G179^{∗}, T38N, N88H, N126Y, T129I, N134M, F153W, L171R, T180D, E187P, I203Y, G476K, G477E, Y303D.
(i) variants exhibiting at least 85% identity with AmyE from *Bacillus subtilis* (SEQ ID NO:1 in WO2009149271).
(j) variants exhibiting at least 90% identity with the wild-type amylase from Bacillus sp. KSM-K38 with accession number AB051102.
(k) variants described in WO2016180748, especially those exhibiting at least 80% identity with the mature amino acid sequence of AAI10 from *Bacillus sp* in SEQ ID NO: 7 in WO2016180748; those exhibiting at least 80% identity with the mature amino acid sequence of *Alicyclobacillus sp.* amylase in SEQ ID NO: 8 in WO2016180748, and those exhibiting at least 80% identity with the mature amino acid sequence of SEQ ID NO: 13 in WO2016180748, especially those comprising one or more of the following mutations H^{∗}, N54S, V56T, K72R, G109A, F113Q, R116Q, W167F, Q172G, A174S, G184T, N195F, V206L, K391A, P473R, G476K.
(l) variants described in WO2018060216, especially those exhibiting at least 70% identity with the mature amino acid sequence of SEQ ID NO: 4 in WO2018060216, the fusion molecule of *Bacillus amyloliquefaciens* and *Bacillus licheniformis.* Especially those comprising one or more substitutions at positions H1, N54, V56, K72, G109, F113, R116, T134, W140, W159, W167, Q169, Q172, L173, A174, R181, G182, D183, G184, W189, E194, N195, V206, G255, N260, F262, A265, W284, F289, S304, G305, W347, K391, Q395, W439, W469, R444, F473, G476, and G477.

Preferred amylases are engineered enzymes, wherein one or more of the amino acids prone to bleach oxidation have been substituted by an amino acid less prone to oxidation. In particular it is preferred that methionine residues are substituted with any other amino acid. In particular it is preferred that the methionine most prone to oxidation is substituted. Preferably the methionine in a position equivalent to 202 in SEQ ID NO:11 is substituted. Preferably, the methionine at this position is substituted with threonine or leucine, preferably leucine.

Suitable commercially available alpha-amylases include DURAMYL^{®}, LIQUEZYME^{®}, TERMAMYL^{®}, TERMAMYL ULTRA^{®}, NATALASE^{®}, SUPRAMYL^{®}, STAINZYME^{®}, STAINZYME PLUS^{®}, FUNGAMYL^{®}, ATLANTIC^{®}, ACHIEVE ALPHA^{®}, AMPLIFY^{®} PRIME, INTENSA^{®} and BAN^{®} (Novozymes A/S, Bagsvaerd, Denmark), KEMZYM^{®} AT 9000 Biozym Biotech Trading GmbH Wehlistrasse 27b A-1200 Wien Austria, RAPIDASE^{®} , PURASTAR^{®}, ENZYSIZE^{®}, OPTISIZE HT PLUS^{®}, POWERASE^{®}, PREFERENZ S^{®} series (including PREFERENZ S1000^{®} and PREFERENZ S2000^{®} and PURASTAR OXAM^{®} (DuPont., Palo Alto, California) and KAM^{®} (Kao, 14-10 Nihonbashi Kayabacho, 1-chome, Chuoku Tokyo 103-8210, Japan).

Preferably, the composition comprises at least 0.01 mg, preferably from about 0.05 to about 10, more preferably from about 0.1 to about 6, especially from about 0.2 to about 5 mg of active amylase/ g of composition.

Peroxidases/Oxidases: Suitable peroxidases/oxidases include those of plant, bacterial or fungal origin. Chemically modified or protein engineered mutants are included. Examples of useful peroxidases include peroxidases from *Coprinus,* e.g., from C. *cinereus,* and variants thereof as those described in WO 93/24618, WO 95/10602, and WO 98/15257.

Commercially available peroxidases include GUARDZYME^{®} (Novozymes A/S).

Pectate lyase: Suitable pectate lyases include those sold under the tradenames Pectawash^{®}, Pectaway^{®}, X-Pect^{®}, (all Novozymes A/S, Bagsvaerd, Denmark) Preferenz^{®} F1000 (DuPont Industrial Biosciences).

Mannanases. The composition preferably comprises one of more mannanase enzymes. As used herein, the term "mannanase" or "galactomannanase" denotes a mannanase enzyme defined according to that known in the art as mannan endo-1,4-beta-mannosidase and having the alternative names beta-mannanase and endo-1,4-mannanase and catalysing hydrolysis of 1,4-beta-D-mannosidic linkages in mannans, galactomannans, glucomannans, and galactoglucomannans. Mannanases are classified according to the Enzyme Nomenclature as EC 3.2.1.78 and belong in Glycosyl Hydrolase families 5, 26 and 113. Many suitable mannanases belong to Glycosyl Hydrolase family 5. Commercially available mannanases include all those sold under the tradenames Mannaway^{®} (Novozymes A/S) such as Mannaway^{®} 200L and Mannaway Evity 4.0T Other commercially available mannanases include Effectenz^{®} M1000, Mannastar^{®} 375, Preferenz M100 and Purabrite^{®} (all DuPont Industrial Biosciences) and Biotouch M7 (AB Enzymes). Other suitable mannanases belong to Glycosyl Hydrolase family 26 including those described in WO2018191135, WO2015040159, WO2017021515, WO2017021516, WO2017021517 and WO2019081515. Suitable mixtures of mannanases include the combinations of Glycosyl Hydrolase family 5 and Glycosyl Hydrolase family 26 mannanases described in WO2019081515.
Nucleases: Preferably the composition comprises a nuclease enzyme such as a RNase or DNase or mixtures thereof. The nuclease enzyme is an enzyme capable of cleaving the phosphodiester bonds between the nucleotide sub-units of nucleic acids. The nuclease enzyme herein is preferably a deoxyribonuclease or ribonuclease enzyme or a functional fragment thereof. By functional fragment or part is meant the portion of the nuclease enzyme that catalyzes the cleavage of phosphodiester linkages in the DNA backbone and so is a region of said nuclease protein that retains catalytic activity. Thus, it includes truncated, but functional versions, of the enzyme and/or variants and/or derivatives and/or homologues whose functionality is maintained.

Preferably the nuclease enzyme is a deoxyribonuclease, preferably selected from any of the classes E.C. 3.1.21.x, where x=1, 2, 3, 4, 5, 6, 7, 8 or 9, E.C. 3.1.22.y where y=1, 2, 4 or 5, E.C. 3.1.30.z where z= 1 or 2, E.C. 3.1.31.1 and mixtures thereof.

DNase: Suitable DNases include wild-types and variants of DNases defined by SEQ ID NOS: 1, 2, 3, 4, 5, 6, 7, 8 and 9 in WO2017162836 (Novozymes), and SEQ ID NOS: 1, 2, 3, 4, 5, 6, 7, 8, 9, 10, 11, 12, 13, 14, 15, 16, 17, 18, 19, 20, 21, 22, 23, 24, 25, 28, 29, 30, 31, 32, 33, 34, 35, 36, 37, 38 39, 40, 41, 42, 43, 44, 45, 46, 47, 48, 49 50, 51, 52, 53, and 54 in WO2018108865, and variants of the Bacillus cibi DNase including those described in WO2018011277 (Novozymes), incorporated herein by reference. Preferred DNases are as claimed in EP3476935A.

RNase: suitable RNases include wild-types and variants of DNases defined by SEQ ID NOS: 3, 6, 9, 12, 15, 57, 58, 59, 60, 61, 62, 63, 64, 65, 66, 67, 72 and 73 in WO2018178061 (Novozymes), and SEQ ID NOS: 86, 87, 88, 89, 90, 91, 92, 93, 94, 95, 96, 97, 98, 99, 100, 101, 102, 103 and 104 in WO2020074499 (Novozymes), incorporated herein by reference.

Hexosaminidase: The composition preferably additionally comprises one or more hexosaminidase enzymes. The term hexosaminidase includes "dispersin" and the abbreviation "Dsp", which means a polypeptide having hexosaminidase activity. Suitable enzymes are found in EC 3.2.1.- that catalyze the hydrolysis of β-1,6-glycosidic linkages of N-acetyl-glucosamine polymers found in soils of microbial origin. The term hexosaminidase includes polypeptides having N-acetylglucosaminidase activity and β-N-acetylglucosaminidase activity. Hexosaminidase activity may be determined according to Assay II described in WO2018184873. Suitable hexosaminidases include those disclosed in WO2017186936, WO2017186937, WO2017186943, WO2017207770, WO2018184873, WO2019086520, WO2019086528, WO2019086530, WO2019086532, WO2019086521, WO2019086526, WO2020002604, WO2020002608, WO2020007863, WO2020007875, WO2020008024, WO2020070063, WO2020070249, WO2020088957, WO2020088958 and WO2020207944. Variants of the *Terribacillus saccharophilus* hexosaminidase defined by SEQ ID NO: 1 of WO2020207944 are preferred, especially the variants with improved thermostability disclosed in that publication.

Particularly preferred hexosaminidase enzymes are hexosaminidase enzymes having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 59; and hexosaminidase enzymes having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 60 herein; and mixtures thereof.

Xanthan gum-degrading enzymes: The composition may comprise one of more xanthan gum-degrading enzymes. Suitable enzymes for degradation of xanthan gum-based soils include xanthan endoglucanase, optionally in conjunction with a xanthan lyase. As used herein, the term "xanthan endoglucanase" denotes an enzyme exhibiting endo-β-1,4-glucanase activity that is capable of catalysing hydrolysis of the 1,4-linked β-D-glucose polymeric backbone of xanthan gum, optionally in conjunction with a suitable xanthan lyase enzyme. Suitable xanthan endoglucanases are described in WO2013167581, WO2015181299, WO2015181292, WO2017046232, WO2017046260, WO201837062, WO201837065, WO2019038059 and WO2019162000. As used herein, the term "xanthan lyase" denotes an enzyme that cleaves the β-D-mannosyl-β-D-1,4-glucuronosyl bond of xanthan gum. Such enzymes belong to E.C. 4.2.2.12. Suitable xanthan lyases are described in WO2015001017, WO2018037061, WO201837064, WO2019038060, WO2019162000 and WO2019038057.

Galactanase: Preferably the composition comprises a galactanase, ie. an extracellular polymer-degrading enzyme that includes an endo-beta-1,6-galactanase enzyme. The term "endo-beta-1,6-galactanase" or "a polypeptide having endo-beta-1,6-galactanase activity" means a endo-beta-1,6-galactanase activity (EC 3.2.1.164) from the glycoside hydrolase family 30 that catalyzes the hydrolytic cleavage of 1,6-3-D-galactooligosaccharides with a degree of polymerization (DP) higher than 3, and their acidic derivatives with 4-O-methylglucosyluronate or glucosyluronate groups at the non-reducing terminals. For purposes of the present disclosure, endo-beta-1,6-galactanase activity is determined according to the procedure described in WO 2015185689 in Assay I. Suitable examples from class EC 3.2.1.164 are described in WO 2015185689, such as the mature polypeptide SEQ ID NO: 2.

The additional enzyme(s) may be included in the detergent composition by adding separate enzyme additives containing an additional enzyme, or a combined enzyme additive comprising two or several or all of the additional enzymes. Such an enzyme additive can be in the form of a granulate, a liquid or slurry, preferably additionally comprising an enzyme stabiliser.

Preferably the or each additional enzyme will be present in the composition in an amount of at least 0.0001 to about 0.1% weight percent of pure active enzyme protein, such as from about 0.0001% to about 0.01%, from about 0.001% to about 0.01% or from about 0.001% to about 0.01% based on the weight of the composition.

Fabric Hueing Agent. The composition may comprise a fabric hueing agent (sometimes referred to as shading, bluing or whitening agents/dyes). Typically the hueing agent provides a blue or violet shade to fabric. Hueing agents can be used either alone or in combination to create a specific shade of hueing and/or to shade different fabric types. This may be provided for example by mixing a red and green-blue dye to yield a blue or violet shade. Hueing agents may be selected from any known chemical class of dye, including but not limited to acridine, anthraquinone (including polycyclic quinones), azine, azo (e.g., monoazo, disazo, trisazo, tetrakisazo, polyazo), including premetallized azo, benzodifurane and benzodifuranone, carotenoid, coumarin, cyanine, diazahemicyanine, diphenylmethane, formazan, hemicyanine, indigoids, methane, naphthalimides, naphthoquinone, nitro and nitroso, oxazine, phthalocyanine, pyrazoles, stilbene, styryl, triarylmethane, triphenylmethane, xanthenes and mixtures thereof. Preferred are azo dyes, especially mono- or bis- azo dyes, triarylmethane dyes and anthraquinone dyes.

Suitable fabric hueing agents include dyes, dye-clay conjugates, and organic and inorganic pigments. Suitable dyes include small molecule dyes and polymeric dyes. Suitable small molecule dyes include small molecule dyes selected from the group consisting of dyes falling into the Colour Index (C.I.) classifications of Direct, Basic, Reactive or hydrolysed Reactive, Solvent or Disperse dyes. Examples of suitable small molecule dyes include for example small molecule dyes selected from the group consisting of Colour Index (Society of Dyers and Colourists, Bradford, UK) numbers Direct Violet dyes such as 9, 35, 48, 51, 66, and 99, Direct Blue dyes such as 1, 71, 80 and 279, Acid Red dyes such as 17, 73, 52, 88 and 150, Acid Violet dyes such as 15, 17, 24, 43, 49, 50 and 51, Acid Blue dyes such as 15, 17, 25, 29, 40, 45, 75, 80, 83, 90 and 113, Acid Black dyes such as 1, Basic Violet dyes such as 1, 3, 4, 10 and 35, Basic Blue dyes such as 3, 16, 22, 47, 66, 75 and 159, Disperse or Solvent dyes such as those described in EP1794275 orEP1794276, or dyes as disclosed in US 7,208,459 B2,and mixtures thereof.

Preferred are polymeric dyes include polymeric dyes selected from the group consisting of polymers containing covalently bound (sometimes referred to as conjugated) chromogens, (dye-polymer conjugates), for example polymers with chromogens co-polymerized into the backbone of the polymer and mixtures thereof. Polymeric dyes include those described in WO2011/98355, WO2011/47987, US2012/090102, WO2010/145887, WO2006/055787 and WO2010/142503.

Preferred polymeric dyes comprise alkoxylated, preferably ethoxylated azo, anthraquinone or triarylmethane dyes. Ethoxylated thiophene azo dyes are especially preferred, for example polymeric dyes selected from the group consisting of fabric-substantive colorants sold under the name of Liquitint^{®} (Milliken, Spartanburg, South Carolina, USA), dye-polymer conjugates formed from at least one reactive dye and a polymer selected from the group consisting of polymers comprising a moiety selected from the group consisting of a hydroxyl moiety, a primary amine moiety, a secondary amine moiety, a thiol moiety and mixtures thereof. Suitable polymeric dyes include polymeric dyes selected from the group consisting of Liquitint^{®} Violet CT, carboxymethyl cellulose (CMC) covalently bound to a reactive blue, reactive violet or reactive red dye such as CMC conjugated with C.I. Reactive Blue 19, sold by Megazyme, Wicklow, Ireland under the product name AZO-CM-CELLULOSE, product code S-ACMC, alkoxylated triphenylmethane polymeric colourants, alkoxylated thiophene polymeric colourants, and mixtures thereof.

Preferred hueing dyes include the alkoxylated thiophene azo whitening agents found in US2008/0177090 which may be optionally anionic, such as those selected from Examples 1-42 in Table 5 of WO2011/011799. Other preferred dyes are disclosed in US 8138222.

Suitable pigments include pigments selected from the group consisting of Ultramarine Blue (C.I. Pigment Blue 29), Ultramarine Violet (C.I. Pigment Violet 15) and mixtures thereof. Pigments and/or dyes may also be added to add colour for aesthetic reasons. Preferred are organic blue, violet and/or green pigments.

Builders: The detergent composition may further contain builders, such as builders based on carbonate, bicarbonate or silicates which may be Zeolites, such as Zeolite A, Zeolite MAP (Maximum Aluminium type P). Zeolites, useable in laundry preferably has the formula Na₁₂(AlO₂)₁₂(SiO₂)₁₂-27H₂O and the particle size is usually between 1-10 µm for zeolite A and 0.7-2 um for zeolite MAP. Other builders are Sodium metasilicate (Na₂SiO₃ · *n*H₂O or Na₂Si₂O₅ · *n* H₂O) strong alkaline and preferably used in dish wash. In preferred embodiments, the amount of a detergent builder may be above 5%, above 10%, above 20%, above 30%, above 40% or above 50%, and may be below 80%, 65%. In a dishwash detergent, the level of builder is typically 40-65%, particularly 50-65% or even 75-90%.

Encapsulates: The composition may comprise an encapsulated benefit agent, comprising a core and a shell having an inner and outer surface, said shell encapsulating said core. The core may comprise a material selected from the group consisting of perfumes; brighteners; dyes; insect repellants; silicones; waxes; flavors; vitamins; fabric softening agents; skin care agents in one aspect, paraffins; enzymes; anti-bacterial agents; bleaches; sensates; and mixtures thereof. The shell may comprise a material selected from the group consisting of polyethylenes; polyamides; polystyrenes; polyisoprenes; polycarbonates; polyesters; polyacrylates; aminoplasts, in one aspect said aminoplast may comprise a polyureas, polyurethane, and/or polyureaurethane, in one aspect said polyurea may comprise polyoxymethyleneurea and/or melamine formaldehyde; polyolefins; polysaccharides, in one aspect said polysaccharide may comprise alginate and/or chitosan; gelatin; shellac; epoxy resins; vinyl polymers; water insoluble inorganics; silicone; and mixtures thereof. Preferred encapsulates comprise a core comprising perfume. Such encapsulates are perfume microcapsules.

Enzyme stabilizer: The composition may comprise an enzyme stabilizer. Suitable enzyme stabilisers may be selected from the group consisting of (a) inorganic salts selected from the group consisting of calcium salts, magnesium salts and mixtures thereof; (b) carbohydrates selected from the group consisting of oligosaccharides, polysaccharides and mixtures thereof and sugar or sugar alcohol; (c) mass efficient reversible protease inhibitors selected from the group consisting of phenyl boronic acid and derivatives thereof, e.g., an aromatic borate ester, or a phenyl boronic acid derivative such as 4-formylphenyl boronic acid, or a peptide aldehyde such as di-, tri- or tetrapeptide aldehydes or aldehyde analogues (either of the form B1-B0-R wherein, R is H, CH3, CX3, CHX2, or CH2X (X=halogen), B0 is a single amino acid residue (preferably with an optionally substituted aliphatic or aromatic side chain); and B1 consists of one or more amino acid residues (preferably one, two or three), optionally comprising an N-terminal protection group, or as described in WO09118375, WO98/13459); and (d) reversible protease inhibitors such as a boron containing compound; (e) polyols such as propylene glycol or glycerol 1-2 propane diol; (f) calcium formate and/or sodium formate; (g) protease inhibitor of the protein type such as RASI, BASI, WASI (bifunctional alpha-amylase/subtilisin inhibitors of rice, barley and wheat) or CI2 or SSI and (h) any combination thereof.

Structurant: In one aspect, the composition may comprise a structurant selected from the group consisting of diglycerides and triglycerides, ethylene glycol distearate microcrystalline cellulose, cellulose-based materials, microfiber cellulose, biopolymers, xanthan gum, gellan gum, and mixtures thereof.
Polymers: The composition preferably comprises one or more polymers. Preferred examples are carboxymethylcellulose, poly(vinyl-pyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid co-polymers and amphiphilic polymers and mixtures thereof.
Amphiphilic cleaning polymers: Preferably, the amphiphilic cleanimg polymer is a compound having the following general structure: bis((C₂H₅O)(C₂H₄O)n)(CH₃)-N⁺-CₓH₂ₓ-N⁺-(CH₃)-bis((C₂H₅O)(C₂H₄O)n), wherein n = from 20 to 30, and x = from 3 to 8, or sulphated or sulphonated variants thereof.

Amphiphilic alkoxylated grease cleaning polymers of the present invention refer to any alkoxylated polymer having balanced hydrophilic and hydrophobic properties such that they remove grease particles from fabrics and surfaces. Specific embodiments of the amphiphilic alkoxylated grease cleaning polymers of the present invention comprise a core structure and a plurality of alkoxylate groups attached to that core structure. These may comprise alkoxylated polyalkylenimines, preferably having an inner polyethylene oxide block and an outer polypropylene oxide block.

The core structure may comprise a polyalkylenimine structure comprising, in condensed form, repeating units of formulae (I), (II), (III) and (IV): 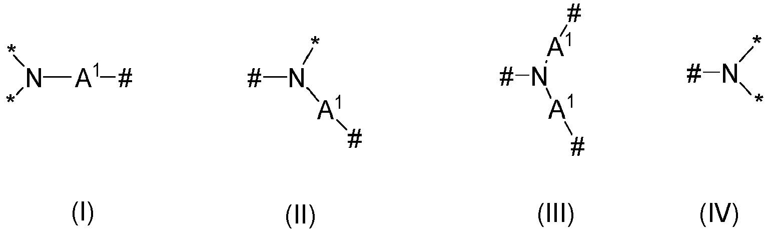 wherein # in each case denotes one-half of a bond between a nitrogen atom and the free binding position of a group A¹ of two adjacent repeating units of formulae (I), (II), (III) or (IV); ^{∗} in each case denotes one-half of a bond to one of the alkoxylate groups; and A¹ is independently selected from linear or branched C₂-C₆-alkylene; wherein the polyalkylenimine structure consists of 1 repeating unit of formula (I), x repeating units of formula (II), y repeating units of formula (III) and y+1 repeating units of formula (IV), wherein x and y in each case have a value in the range of from 0 to about 150; where the average weight average molecular weight, Mw, of the polyalkylenimine core structure is a value in the range of from about 60 to about 10,000 g/mol.

The core structure may alternatively comprise a polyalkanolamine structure of the condensation products of at least one compound selected from N-(hydroxyalkyl)amines of formulae (I.a) and/or (I.b), 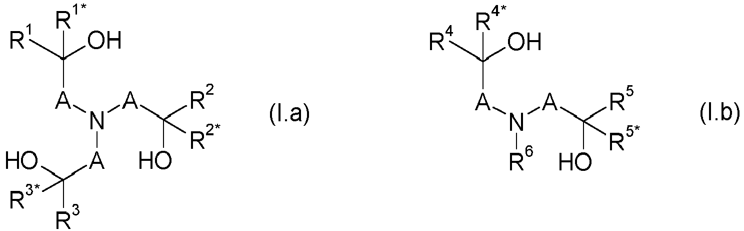 wherein A are independently selected from C₁-C₆-alkylene; R¹, R^{1∗}, R², R^{2∗}, R³, R^{3∗}, R⁴, R^{4∗}, R⁵ and R^{5∗} are independently selected from hydrogen, alkyl, cycloalkyl or aryl, wherein the last three mentioned radicals may be optionally substituted; and R⁶ is selected from hydrogen, alkyl, cycloalkyl or aryl, wherein the last three mentioned radicals may be optionally substituted.

The plurality of alkylenoxy groups attached to the core structure are independently selected from alkylenoxy units of the formula (V) 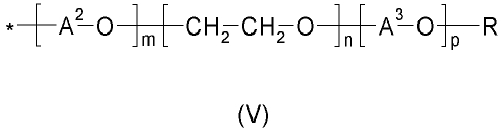 wherein ^{∗} in each case denotes one-half of a bond to the nitrogen atom of the repeating unit of formula (I), (II) or (IV); A² is in each case independently selected from 1,2-propylene, 1,2-butylene and 1,2-isobutylene; A³ is 1,2-propylene; R is in each case independently selected from hydrogen and C₁-C₄-alkyl; m has an average value in the range of from 0 to about 2; n has an average value in the range of from about 20 to about 50; and p has an average value in the range of from about 10 to about 50.

Carboxylate polymer: The composition preferably also includes one or more carboxylate polymers such as a maleate/acrylate random copolymer or polyacrylate homopolymer. In one aspect, the carboxylate polymer is a polyacrylate homopolymer having a molecular weight of from 4,000 Da to 9,000 Da, or from 6,000 Da to 9,000 Da.

Soil release polymer: The composition preferably also comprises one or more soil release polymers having a structure as defined by one of the following structures (I), (II) or (III):
(I)

   -[(OCHR¹-CHR²)ₐ-O-OC-Ar-CO-]_{d}
(II)

   -[(OCHR³-CHR⁴)_{b}-O-OC-sAr-CO-]ₑ
(III)

   -[(OCHR⁵-CHR⁶)_{c}-OR7]^{f}
wherein:
a, b and c are from 1 to 200;
d, e and f are from 1 to 50;
Ar is a 1,4-substituted phenylene;
sAr is 1,3-substituted phenylene substituted in position 5 with SO₃Me;
Me is Li, K, Mg/2, Ca/2, Al/3, ammonium, mono-, di-, tri-, or tetraalkylammonium wherein the alkyl groups are C₁-C₁₈ alkyl or C₂-C₁₀ hydroxyalkyl, or mixtures thereof;
R¹, R², R³, R⁴, R⁵ and R⁶ are independently selected from H or C₁-C₁₈ n- or iso-alkyl; and
R⁷ is a linear or branched C₁-C₁₈ alkyl, or a linear or branched C₂-C₃₀ alkenyl, or a cycloalkyl group with 5 to 9 carbon atoms, or a C₈-C₃₀ aryl group, or a C₆-C₃₀ arylalkyl group.

Suitable soil release polymers are polyester soil release polymers such as Repel-o-tex polymers, including Repel-o-tex SF, SF-2 and SRP6 supplied by Rhodia. Other suitable soil release polymers include Texcare polymers, including Texcare SRA100, SRA300, SRN100, SRN170, SRN240, SRN300 and SRN325 supplied by Clariant. Other suitable soil release polymers are Marloquest polymers, such as Marloquest SL supplied by Sasol.

Cellulosic polymer: The composition preferably also comprises one or more cellulosic polymers including those selected from alkyl cellulose, alkyl alkoxyalkyl cellulose, carboxyalkyl cellulose, alkyl carboxyalkyl cellulose. In one aspect, the cellulosic polymers are selected from the group comprising carboxymethyl cellulose, methyl cellulose, methyl hydroxyethyl cellulose, methyl carboxymethyl cellulose, and mixures thereof. In one aspect, the carboxymethyl cellulose has a degree of carboxymethyl substitution from 0.5 to 0.9 and a molecular weight from 100,000 Da to 300,000 Da.

Bleaching system: The composition may contain a bleaching system, for example comprising a H₂O₂ source such as perborate or percarbonate which may be combined with a peracid-forming bleach activator such as tetraacetylethylenediamine or nonanoyloxybenzenesulfonate. Alternatively, the bleaching system may comprise peroxyacids of, e.g., the amide, imide, or sulfone type. In general, when a bleaching agent is used, the compositions of the present invention may comprise from about 0.1% to about 30% or even from about 0.1 % to about 25% bleaching agent by weight of the subject cleaning composition.

Chelating Agents: The composition preferably comprises a chelating agent, preferably in an amount from 0.005% to about 15% or even from about 3.0% to about 10% chelating agent by weight of the composition. Suitable chelating agents include copper, iron and/or manganese chelating agents and mixtures thereof. Preferred chelants (complexing agents) include DTPA (Diethylene triamine pentaacetic acid), HEDP (Hydroxyethane diphosphonic acid), DTPMP (Diethylene triamine penta(methylene phosphonic acid)), 1,2-Dihydroxybenzene-3,5-disulfonic acid disodium salt hydrate, ethylenediamine, diethylene triamine, ethylenediaminedisuccinic acid (EDDS), N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP), methyl-glycine-diacetic acid (MGDA), glutamic-N,N- diacetic acid (GLDA), iminodisuccinic acid (IDS), carboxy methyl inulin; and salts derivatives thereof and mixtures thereof. Preferred chelants are selected from the group consisting of methyl-glycine-diacetic acid (MGDA), its salts and derivatives thereof, glutamic-N,N- diacetic acid (GLDA), its salts and derivatives thereof, iminodisuccinic acid (IDS), its salts and derivatives thereof, carboxy methyl inulin, its salts and derivatives thereof and mixtures thereof. MGDA and salts thereof are especially preferred, in particular comprising the three-sodium salt of MGDA.

The composition may also contain other conventional detergent ingredients such as e.g. fabric conditioners including clays, foam boosters, suds suppressors, anti-corrosion agents, soil-suspending agents, anti-soil re-deposition agents, dyes, bactericides, optical brighteners, hydrotropes, tarnish inhibitors, organic solvents such as ethanol or perfumes.

### Method of Use

The present invention also provides a method for treating a fabric, the method comprising in a contacting step, contacting a fabric with an aqueous wash liquor comprising an alginate lyase enzyme as described above, preferably in an amount from O.Olppm to 10ppm, preferably from 0.1ppm to 1ppm; and booster enzyme as described above preferably in an amount from O.Olppm to 10ppm, preferably from 0.1ppm to 1ppm; and preferably additionally an anionic surfactant, preferably in an amount from 0.05 to 50g/l, more preferably from 0.2g/l to 5g/l or 0.5g/l to 3g/l.

The aqueous wash liquor may be formed by adding a composition as described above to water, for example in a washing machine or hand washing process. Alternatively, the aqueous wash liquor may be formed by adding the alginate lyase enzyme, booster enzyme and any cleaning adjunct as separate components, into water to form the wash liquor. The fabric may be optionally subsequently washed, and/or rinsed and/or dried.

The alginate lyase enzyme, booster enzyme and any additional enzyme may be present in the wash liquor in an amount corresponding to 0.001-100 mg of active enzyme protein per liter of wash liquor, preferably 0.005-5 mg of active enzyme protein per liter of wash liquor, more preferably 0.01-1 mg of enzyme protein per liter of wash liquor and in particular 0.1-1 mg of enzyme protein per liter of wash.

In the contacting step, or in a subsequent step, it may be preferred to use mechanical agitation to promote cleaning and removal of the broken-down soil by-products from the fabric. The wash liquor preferably has a pH of from about 7 or 8 to about 10.5. The composition may typically be employed at concentrations of from about 500 ppm to about 15,000 ppm in solution to form the wash liquor. The wash liquor preferably has a temperature from about 5 °C to about 40 °C, or preferably from 10 to 35 °C or 30 °C or 25 °C. The water to fabric ratio is typically from about 1:1 to about 30:1.

### TEST METHODS

### Test Method 1

### Enzymatic activity towards β-D-mannuronic acid blocks (polyM activity) and α-L-guluronic acid blocks (polyG activity)

The alginate lyase activity is measured using Mannuronic Block Oligosaccharide DP20-DP35 (Product code: ALG601) and Guluronate oligosaccharide DP2-DP45 (Product code: ALG610) from Elicityl, France as substrates. Mannuronic Block Oligosaccharide DP20-DP35 is used to measure polyM activity, whereas Guluronate oligosaccharide DP2-DP45 was used for measuring polyG activity.

A solution of 2.5% of each substrate was suspended in Tris buffer at pH 8.3 and incubated with 3 ppm of each alginate lyase of interest, at 25°C for 60 min, in a 96 well plate.

The activity towards each of the substrates is given as the delta absorbance at 235nm in a spectrophotometer vs nil enzyme sample when the enzyme was put in contact with each of the substrates. These values are then used to evaluate the activity towards β-D-mannuronic acid blocks (polyM) and/or α-L-guluronic acid blocks (polyG) of the respective enzymes. An enzyme having activity towards poly(beta-D-mannuronate) (polyM activity) preferably provides a delta absorbance versus nil enzyme of at least 0.1 absorbance units, more preferably at least 0.15 absorbance units and more preferably at least 0.2 absorbance units. An enzyme having activity towards poly(alpha-L-guluronate) (polyG activity) preferably provides a delta absorbance versus nil enzyme of at least 0.3 absorbance units, preferably at least 0.4 or even 0.5 or 0.6 absorbance units.

### Test Method 2

### Measurement of activity against beta-1,3-glucan and beta-1,3(4)-glucan

Activity against beta-1,3-glucan and beta-1,3(4)-glucan was measured using the red variant of the Discovery kit supplied by GlycoSpot A/S, Søborg, Denmark which allows simultaneous assay of a given enzyme across 16 different activities. Protocol for the assay is given below: if a given enzyme shows greater than 0.3 absorbance units against the curdlan (CPH0009) AND beta glucan from barley (CPH0003) it is deemed to have significant activity towards both beta-1,3-glucan and beta-1,3(4)-glucan.
1. Add 200 µl activation solution to each well in the 96-well plate.
2. Incubate 10 minutes at room temperature without agitation.
3. Remove the remaining solution by centrifugation (2700 xg for 10 min or vacuum).
4. Wash twice with 100 µl water to remove the stabiliser completely.
5. Add samples and controls to each well in the 96-well plate: The controls are 150 µl 1M tris(hydroxymethyl)aminomethane hydrochloride (Tris-HCL) buffer (pH 8). The samples are 1mg of active enzyme protein dissolved in 150 µl of the same Tris-HCL buffer.
6. Put a 'product plate' underneath the substrate plate.
7. Incubate the reaction at 30°C for 30 minutes.
8. Transfer the reaction product into the product plate by centrifugation (2700 xg for 10 min or vacuum).
9. Check that the volume in each well is equal.
10. Detect the absorbance (517 nm).

### DETERGENT EXAMPLES

### Examples 1-6. Granular laundry detergent compositions designed for hand washing or top-loading washing machines.

| | 1 (wt %) | 2 (wt %) | 3 (wt %) | 4 (wt %) | 5 (wt %) | 6 (wt %) |
|---|---|---|---|---|---|---|
| Linear alkylbenzenesulfonate | 20 | 22 | 20 | 15 | 20 | 20 |
| C12-14 Dimethylhydroxyethyl ammonium chloride | 0.7 | 0.2 | 1 | 0.6 | 0.0 | 0.0 |
| AE3S | 0.9 | 1 | 0.9 | 0.0 | 0.5 | 0.9 |
| AE7 | 0.0 | 0.0 | 0.0 | 1 | 0.0 | 3 |
| Sodium tripolyphosphate | 5 | 0.0 | 4 | 9 | 2 | 0.0 |
| Zeolite A | 0.0 | 1 | 0.0 | 1 | 4 | 1 |
| 1.6R Silicate (SiO2:Na2O at ratio 1.6:1) | 7 | 5 | 2 | 3 | 3 | 5 |
| Sodium carbonate | 25 | 20 | 25 | 17 | 18 | 19 |
| Polyacrylate MW 4500 | 1 | 0.6 | 1 | 1 | 1.5 | 1 |
| Random graft copolymer¹ | 0.1 | 0.2 | 0.0 | 0.0 | 0.0 | 0.0 |
| Carboxymethyl cellulose | 1 | 0.3 | 1 | 1 | 1 | 1 |
| Protease (Savinase^{®}, 32.89 mg active/g) | 0.1 | 0.1 | 0.1 | 0.1 | | 0.1 |
| ⁵DNase as defined herein (mg active per 100g composition) | 2.0 | 0 | 4.4 | 0 | 0.4 | 0 |
| Lipase - Lipex^{®} (18 mg active /g) | 0.03 | 0.07 | 0.3 | 0.1 | 0.07 | 0.4 |
| ⁴Amylase Stainzyme^{®} Plus (mg active) | 3.0 | 5.0 | 3.0 | 2.2 | 6.0 | 6.0 |
| ⁶Alginate lyase as defined herein (mg active per 100g of detergent) | 12.0 | 15.0 | 3.2 | 4.3 | 9.2 | 17.0 |
| ⁷Hexosaminidase as defined herein (mg active per 100g composition) | 2.0 | 6.0 | 5.6 | 2.2 | 4.0 | 1.5 |
| ⁸Pel-ase as defined herein (mg active per 100g composition) | 4.3 | 0.0 | 3.4 | 7.2 | 1.9 | 3.3 |
| ⁹Psl-ase as defined herein (mg active per 100g composition) | 3.2 | 3.2 | 0.0 | 1.2 | 0.0 | 5.3 |
| ¹⁰Endo-beta-1,3(4)-glucanase as defined herein (mg active per 100g composition) | 9.1 | 5.4 | 0.0 | 4.3 | 3.0 | 3.1 |
| Fluorescent Brightener 1 | 0.06 | 0.0 | 0.06 | 0.18 | 0.06 | 0.06 |
| Fluorescent Brightener 2 | 0.1 | 0.06 | 0.1 | 0.0 | 0.1 | 0.1 |
| DTPA | 0.6 | 0.8 | 0.6 | 0.25 | 0.6 | 0.6 |
| MgSO4 | 1 | 1 | 1 | 0.5 | 1 | 1 |
| Sodium Percarbonate | 0.0 | 5.2 | 0.1 | 0.0 | 0.0 | 0.0 |
| Sodium Perborate Monohydrate | 4.4 | 0.0 | 3.85 | 2.09 | 0.78 | 3.63 |
| NOBS | 1.9 | 0.0 | 1.66 | 0.0 | 0.33 | 0.75 |
| TAED | 0.58 | 1.2 | 0.51 | 0.0 | 0.015 | 0.28 |
| Sulphonated zinc phthalocyanine | 0.0030 | 0.0 | 0.0012 | 0.0030 | 0.0021 | 0.0 |
| S-ACMC | 0.1 | 0.0 | 0.0 | 0.0 | 0.06 | 0.0 |
| Direct Violet 9 | 0.0 | 0.0 | 0.0003 | 0.0005 | 0.0003 | 0.0 |
| Acid Blue 29 | 0.0 | 0.0 | 0.0 | 0.0 | 0.0 | 0.0003 |
| Sulfate/Moisture | Balance | | | | | |

### Examples 7-13. Granular laundry detergent compositions designed for front-loading automatic washing machines.

| | 7 (wt%) | 8 (wt%) | 9 (wt%) | 10 (wt%) | 11 (wt%) | 12 (wt%) | 13 (wt%) |
|---|---|---|---|---|---|---|---|
| Linear alkylbenzenesulfonate | 8 | 7.1 | 7 | 6.5 | 7.5 | 7.5 | 11 |
| AE3S | 0 | 4.8 | 0 | 5.2 | 4 | 4 | 0 |
| C12-14 Alkylsulfate | 1 | 0 | 1 | 0 | 0 | 0 | 1 |
| AE7 | 2.2 | 0 | 3.2 | 0 | 0 | 0 | 1 |
| CI0-12 Dimethyl hydroxyethylammonium chloride | 0.75 | 0.94 | 0.98 | 0.98 | 0 | 0 | 0 |
| Crystalline layered silicate (δ-Na2Si2O5) | 4.1 | 0 | 4.8 | 0 | 0 | 0 | 7 |
| Zeolite A | 5 | 0 | 5 | 0 | 2 | 2 | 4 |
| Citric Acid | 3 | 5 | 3 | 4 | 2.5 | 3 | 0.5 |
| Sodium Carbonate | 15 | 20 | 14 | 20 | 23 | 23 | 14 |
| Silicate 2R (SiO2:Na2O at ratio 2:1) | 0.08 | 0 | 0.11 | 0 | 0 | 0 | 0.01 |
| Soil release agent | 0.75 | 0.72 | 0.71 | 0.72 | 0 | 0 | 0.1 |
| Acrylic Acid/Maleic Acid Copolymer | 1.1 | 3.7 | 1.0 | 3.7 | 2.6 | 3.8 | 2 |
| Carboxymethylcellulose | 0.15 | 1.4 | 0.2 | 1.4 | 1 | 0.5 | 0.2 |
| Protease - Purafect^{®} (84 mg active/g) | 0.2 | 0.2 | 0.3 | 0.15 | 0.12 | 0.13 | 0.18 |
| Lipase - Lipex^{®} (18.00 mg active/g) | 0.05 | 0.15 | 0.1 | 0 | 0 | 0 | 0.1 |
| Cellulase - CellucleanTM (15.6 mg active/g) | 0 | 0 | 0 | 0 | 0.1 | 0.1 | 0 |
| ⁴Amylase Stainzyme^{®} Plus (mg active) | 4.0 | 5.0 | 10 | 2.2 | 4.4 | 1.5 | 1.5 |
| Mannanase - Mannaway^{®} (4mg active/g) | 0.05 | 0.1 | 0 | 0.05 | 0.1 | 0 | 0.1 |
| ⁵DNase as defined herein (mg active per 100g detergent) | 1.0 | 0 | 2.0 | 0 | 4.0 | 0 | 0 |
| ⁶Alginate lyase as defined herein (mg active per 100g of detergent) | 3.3 | 9.2 | 12.0 | 4.7 | 3.7 | 13.2 | 3.3 |
| ⁷Hexosaminidase as defined herein (mg active per 100g detergent) | 3.0 | 5.0 | 8.0 | 2.2 | 4.0 | 1.5 | 0.1 |
| ⁸Pel-ase as defined herein (mg active per 100g composition) | 3.2 | 5.3 | 0.0 | 0.0 | 0.0 | 3.0 | 3.3 |
| ⁹Psl-ase as defined herein (mg active per 100g composition) | 0.0 | 0.0 | 0.0 | 3.2 | 8.2 | 1.1 | 0.9 |
| ¹⁰Endo-beta-1,3(4)-glucanase as defined herein (mg active per 100g composition) | 0.0 | 0.0 | 3.2 | 1.0 | 3.3 | 2.2 | 3.2 |
| TAED | 3.6 | 4.0 | 3.6 | 4.0 | 2.2 | 1.4 | 1 |
| Percarbonate | 13 | 13.2 | 13 | 13.2 | 16 | 14 | 10 |
| Na salt of Ethylenediamine-N,N'-disuccinic acid, (S,S) isomer (EDDS) | 0.2 | 0.2 | 0.001 | 0.2 | 0.2 | 0.2 | 0.001 |
| Hydroxyethane di phosphonate (HEDP) | 0.2 | 0.2 | 0.5 | 0.2 | 0.2 | 0.2 | 0.5 |
| MgSO4 | 0.42 | 0.42 | 0.42 | 0.42 | 0.4 | 0.4 | 0 |
| Perfume | 0.5 | 0.6 | 0.5 | 0.6 | 0.6 | 0.6 | 0.8 |
| Suds suppressor agglomerate | 0.05 | 0.1 | 0.05 | 0.1 | 0.06 | 0.05 | 0.05 |
| Soap | 0.45 | 0.45 | 0.45 | 0.45 | 0 | 0 | 0 |
| Sulphonated zinc phthalocyanine (active) | 0.000 7 | 0.001 2 | 0.000 7 | 0 | 0 | 0 | 0 |
| S-ACMC | 0.01 | 0.01 | 0 | 0.01 | 0 | 0 | 0 |
| Direct Violet 9 (active) | 0 | 0 | 0.000 1 | 0.000 1 | 0 | 0 | 0.001 |
| Sulfate/ Water & Miscellaneous | Balance | | | | | | |

| | | | | | | | |
|---|---|---|---|---|---|---|---|
| ^{∗}DNase is shown as mgs of active enzyme per 100g of detergent. | | | | | | | |

### Examples 14-21. Heavy Duty Liquid laundry detergent compositions

| | 14 (wt%) | 15 (wt%) | 16 (wt%) | 17 (wt%) | 18 (wt%) | 19 (wt%) | 20 (wt%) | 21 (wt%) |
|---|---|---|---|---|---|---|---|---|
| C12-15 Alkylethoxy(1.8)sulfate | 14.7 | 11.6 | 0.0 | 16.3 | 0.0 | 17.3 | 20 | 12 |
| C11.8 Alkylbenzene sulfonate | 4.3 | 11.6 | 8.3 | 7.8 | 11.7 | 7.8 | 7 | 0 |
| C16-17 Branched alkyl sulfate | 1.7 | 1.29 | 0.0 | 3.09 | 0.0 | 3.3 | 0 | 0 |
| C12-14 Alkyl -9-ethoxylate | 0.9 | 1.07 | 0.0 | 1.31 | 0.0 | 1.31 | 5 | 0 |
| C12 dimethylamine oxide | 0.6 | 0.64 | 0.0 | 1.03 | 0.0 | 1.03 | 2 | 3 |
| Citric acid | 3.5 | 0.65 | 3 | 0.66 | 2.27 | 0.67 | 1 | 0 |
| C12-18 fatty acid | 1.5 | 2.32 | 3.6 | 1.52 | 0.82 | 1.52 | 1 | 0 |
| Sodium Borate (Borax) | 2.5 | 2.46 | 1.2 | 2.53 | 0.0 | 2.53 | 0 | 1 |
| Sodium C12-14 alkyl ethoxy 3 sulfate | 0.0 | 0.0 | 2.9 | 0.0 | 3.9 | 0.0 | 0 | 14 |
| C14-15 alkyl 7-ethoxylate | 0.0 | 0.0 | 4.2 | 0.0 | 1.9 | 0.0 | 0 | 4.2 |
| C12-14 Alkyl -7-ethoxylate | 0.0 | 0.0 | 1.7 | 0.0 | 0.5 | 0.0 | 0 | 1.7 |
| Ca chloride dihydrate | 0.0 | 0.0 | 0.0 | 0.0 | 0.045 | 0.0 | 0 | 0 |
| Ca formate | 0.09 | 0.09 | 0.0 | 0.09 | 0.0 | 0.09 | 0.09 | 0 |
| A compound: bis((C2H5O)(C2H4O)n)(C H3)-N+-CxH2x-N+-(CH3)-bis((C2H5O)(C2H4O)n); n is 20 to 30; x is 3 to 8, optionally sulphated or sulphonated | 0.0 | 0.0 | 1.2 | 0.0 | 0.66 | 0.0 | 0.0 | 1.2 |
| Random graft co-polymer¹ | 0.0 | 1.46 | 0.5 | 0.0 | 0.83 | 0.0 | 0.0 | 0.5 |
| Ethoxylated Polyethylenimine ² | 1.5 | 1.29 | 0.0 | 1.44 | 0.0 | 1.44 | 1.44 | 0.0 |
| Diethylene triamine pentaacetic acid | 0.34 | 0.64 | 0.0 | 0.34 | 0.0 | 0.34 | 0.34 | 0.0 |
| Diethylene triamine penta (methylene phosphonic acid) | 0.0 | 0.0 | 0.3 | 0.0 | 0.3 | 0.0 | 0.0 | 0.3 |
| 1-hydroxyethyidene-1,1-diphosphonic acid | 0.0 | 0.0 | 0.0 | 0.0 | 0.18 | 0.0 | 0.0 | 0.0 |
| Dihydroxybenzene-3,5-disulfonic acid disodium salt hydrate | 0.0 | 0.0 | 0.0 | 0.0 | 0.0 | 0.19 | 0.19 | 0.0 |
| Tinopal AMS-GX | 0.0 | 0.06 | 0.0 | 0.0 | 0.0 | 0.29 | 0.29 | 0.0 |
| Tinopal CBS-X | 0.2 | 0.17 | 0.0 | 0.29 | 0.0 | 0.0 | 0.0 | 0.0 |
| Tinopal TAS-X B36 | 0.0 | 0.0 | 0.0 | 0.0 | 0.091 | 0.0 | 0.0 | 0.0 |
| Amphiphilic alkoxylated grease cleaning polymer ³ | 1.28 | 1 | 0.4 | 1.93 | 0.0 | 1.93 | 1.93 | 0.4 |
| CHEC | 0.0 | 0.0 | 0.2 | 0.0 | 0.0 | 0.0 | 0.0 | 0.2 |
| Ethanol | 2 | 1.58 | 1.6 | 5.4 | 1.2 | 3.57 | 0 | 1.6 |
| Propylene Glycol | 3.9 | 3.59 | 1.3 | 4.3 | 0.0 | 3.8 | 3.8 | 1.3 |
| Diethylene glycol | 1.05 | 1.54 | 0.0 | 1.15 | 0.0 | 1.15 | 1.15 | 0.0 |
| Polyethylene glycol | 0.06 | 0.04 | 0.0 | 0.1 | 0.0 | 0.1 | 0.1 | 0.0 |
| ⁴Amylase Amplify^{®} (mg active) | 8.0 | 7.0 | 2.5 | 4.0 | 3.0 | 1.7 | 3 | 2.5 |
| ⁵DNase (mg active per 100g detergent) | 0 | 2.0 | 0 | 2.0 | 1.25 | 1.0 | 5 | 0 |
| ⁶Alginate lyase as defined herein (mg active per 100g of detergent) | 3.2 | 4.1 | 7.9 | 12.4 | 3.7 | 5.0 | 17.3 | 2.1 |
| ⁷Hexosaminidase as defined herein (mg active per 100g detergent) | 7.0 | 3.0 | 8.0 | 2.2 | 1.25 | 10 | 0.1 | 2.5 |
| ⁸Pel-ase as defined herein (mg active per 100g composition) | 2.2 | 0.0 | 1.2 | 0.8 | 0.10 | 8.3 | 2.2 | 3.9 |
| ⁹Psl-ase as defined herein (mg active per 100g composition) | 3.4 | 4.4 | 0.0 | 0.0 | 0.0 | 4.3 | 5.3 | 3.2 |
| ¹⁰Endo-beta-1,3(4)-glucanase as defined herein (mg active per 100g composition) | 2.2 | 0.0 | 1.4 | 5.3 | 2.2 | 3.3 | 4.5 | 3.3 |
| Monoethanolamine | 3.05 | 2.41 | 0.4 | 1.26 | 0.31 | 1.13 | 1.13 | 0.4 |
| NaOH | 2.44 | 1.8 | 0.0 | 3.01 | 3.84 | 0.24 | 0.24 | 0.0 |
| Sodium Cumene Sulphonate | 0.0 | 0.0 | 1 | 0.0 | 0.95 | 0.0 | 0.0 | 1 |
| Sodium Formate | 0.0 | 0.11 | 0.0 | 0.09 | 0.2 | 0.12 | 0.12 | 0.0 |
| Polyethoxylated azo thiophene dye | 0.001 | 0.001 | 0.001 | 0.05 | 0.000 1 | 0.000 1 | 0.000 1 | 0.001 |
| Water, Aesthetics (Dyes, perfumes) and Minors (Enzymes including lipase, protease, additional amylase each at 0.2% active protein, solvents, structurants) | | | | balance | | | | |

### Examples 22-28. Unit Dose Laundry detergent compositions. Such unit dose formulations can comprise one or multiple compartments.

| | 22 (wt%) | 23 (wt%) | 24 (wt%) | 25 (wt%) | 26 (wt%) | 27 (wt%) | 28 (wt%) |
|---|---|---|---|---|---|---|---|
| Alkylbenzene sulfonic acid | 14.5 | 14.5 | 14.5 | 14.5 | 14.5 | 23 | 23 |
| C12-18 alkyl ethoxy 2.5 sulfate | 7.5 | 7.5 | 7.5 | 7.5 | 7.5 | 16 | 16 |
| C12-18 alkyl 7-ethoxylate | 13.0 | 13.0 | 13.0 | 13.0 | 13.0 | 3.1 | 3.8 |
| C14-15 alkyl 9-ethoxylate | 0 | 0 | 0 | 0 | 0 | 1 | 0 |
| Citric Acid | 0.6 | 0.6 | 0.6 | 0.6 | 0.6 | 0.9 | 0.7 |
| Fatty Acid | 14.8 | 14.8 | 14.8 | 14.8 | 14.8 | 6.5 | 6 |
| Amylase (mg active) | 6 | 12 | 8 | 2 | 10 | 2 | 2 |
| Ethoxylated Polyethylenimine² | 4.0 | 4.0 | 4.0 | 4.0 | 4.0 | 4.0 | 4.0 |
| Protease (Purafect Prime^{®}, 40.6 mg active/g) | 1.4 | 2.0 | 0.9 | 1.2 | 0 | 1 | 1 |
| Cellulase (Celluclean, active protein) | 0.1 | 0.2 | 0.0 | 0.0 | 0.1 | 0 | 0 |
| ⁵DNase described herein (mg active per 100g detergent) | 3.0 | 2.0 | 1.0 | 2.0 | 2.0 | 1 | 1 |
| ⁴Amylase Amplify^{®} (active protein) | 0.0 | 0.0 | 0.1 | 0.2 | 0.1 | 0.5 | 0.5 |
| ⁶Alginate lyase as defined herein (mg active per 100g of detergent) | 2.2 | 3.1 | 2.3 | 5.2 | 5.3 | 12.2 | 5.4 |
| ⁷Hexosaminidase as defined herein (mg active per 100g detergent) | 5.0 | 10 | 0.0 | 2.2 | 1.2 | 3 | 0.9 |
| ⁸Pel-ase as defined herein (mg active per 100g composition) | 1.3 | 0.0 | 2.45 | 8.3 | 4.0 | 3.2 | 5.3 |
| ⁹Psl-ase as defined herein (mg active per 100g composition) | 0.0 | 3.4 | 7.3 | 3.2 | 1.5 | 4.7 | 0.0 |
| ¹⁰Endo-beta-1,3(4)-glucanase as defined herein (mg active per 100g composition) | 3.4 | 3.3 | 3.4 | 0.0 | 3.1 | 1.9 | 6.3 |
| Hydroxyethane diphosphonic acid | 1.2 | 1.2 | 1.2 | 1.2 | 1.2 | 0 | 2.3 |
| Brightener | 0.3 | 0.3 | 0.3 | 0.3 | 0.3 | 0.2 | 0.2 |
| P-diol | 15.8 | 13.8 | 13.8 | 13.8 | 13.8 | 12.2 | 12.2 |
| Glycerol | 6.1 | 6.1 | 6.1 | 6.1 | 6.1 | 4.0 | 3.8 |
| MEA | 8.0 | 8.0 | 8.0 | 8.0 | 8.0 | 8.6 | 10.2 |
| TIPA | 0.0 | 0.0 | 2.0 | 0.0 | 0.0 | 0.0 | 0.0 |
| TEA | 0.0 | 2.0 | 0.0 | 0.0 | 0.0 | 0.0 | 0.0 |
| Cumene sulphonate | 0.0 | 0.0 | 0.0 | 0.0 | 2.0 | 0.0 | 0.0 |
| Cyclohexyl dimethanol | 0.0 | 0.0 | 0.0 | 2.0 | 0.0 | 0.0 | 0.0 |
| Water | 10 | 10 | 10 | 10 | 10 | 10 | 10 |
| Structurant | 0.14 | 0.14 | 0.14 | 0.14 | 0.14 | 0.14 | 0.14 |
| Perfume | 1.9 | 1.9 | 1.9 | 1.9 | 1.9 | 1.9 | 1.9 |
| Buffers (monoethanolamine) | To pH 8.0 | | | | | | |
| Solvents (1,2 propanediol, ethanol) & minors | To 100% | | | | | | |

### Example 29. Multiple Compartment Unit Dose Composition

Multiple compartment unit dose laundry detergent formulations of the present invention are provided below. In these examples the unit dose has three compartments, but similar compositions can be made with two, four or five compartments. The film used to encapsulate the compartments is polyvinyl alcohol.

| Base composition 1 | 24 (wt%) |
|---|---|
| Glycerol (min 99) | 5.3 |
| 1,2-propanediol | 10.0 |
| Citric Acid | 0.5 |
| Monoethanolamine | 10.0 |
| Caustic soda | - |
| Dequest 2010 | 1.1 |
| Potassium sulfite | 0.2 |
| ⁵DNase as defined herein (mg active) | 4.0 |
| ⁶Alginate lyase as defined herein (mg active per 100g of detergent) | 12.2 |
| ⁷Hexosaminidase as defined herein (mg active per 100g detergent) | 7.0 |
| ⁸Pel-ase as defined herein (mg active per 100g composition) | 2.3 |
| ⁹Psl-ase as defined herein (mg active per 100g composition) | 4.2 |
| ¹⁰Endo-beta-1,3(4)-glucanase as defined herein (mg active per 100g composition) | 5.4 |
| Nonionic Marlipal C24EO7 | 20.1 |
| HLAS | 24.6 |
| Optical brightener FWA49 | 0.2 |
| C12-15 Fatty acid | 16.4 |
| Polymer Lutensit Z96 | 2.9 |
| Polyethyleneimine ethoxylate PEI600 E20 | 1.1 |
| MgCl2 | 0.2 |
| Solvents (1,2 propanediol, ethanol) | To 100% |

**Multi-compartment formulations**

| Composition | 1 | | | 2 | | |
|---|---|---|---|---|---|---|
| Compartment | A | B | C | A | B | C |
| Volume of each compartment | 40 ml | 5 ml | 5 ml | 40 ml | 5 ml | 5 ml |
| Active material in Wt.% | | | | | | |
| Perfume | 1.6 | 1.6 | 1.6 | 1.6 | 1.6 | 1.6 |
| Dyes | < 0.01 | < 0.01 | < 0.01 | < 0.01 | < 0.01 | < 0.01 |
| TiO2 | 0.1 | - | - | - | 0.1 | - |
| Sodium Sulfite | 0.4 | 0.4 | 0.4 | 0.3 | 0.3 | 0.3 |
| Acusol 305 | 1.2 | | | 2 | - | - |
| Hydrogenated castor oil | 0.14 | 0.14 | 0.14 | 0.14 | 0.14 | 0.14 |
| Base Composition 1 | Add to 100% | Add to 100% | Add to 100% | Add to 100% | Add to 100% | Add to 100% |

Raw Materials and Notes for Composition Examples 1-29
Linear alkylbenzenesulfonate having an average aliphatic carbon chain length C11-C18
C12-18 Dimethylhydroxyethyl ammonium chloride
AE3S is C12-15 alkyl ethoxy (3) sulfate
AE7 is C12-15 alcohol ethoxylate, with an average degree of ethoxylation of 7
AE9 is C12-16 alcohol ethoxylate, with an average degree of ethoxylation of 9
HSAS is a mid-branched primary alkyl sulfate with carbon chain length of about 16-17 as disclosed in US 6,020,303 and US 6,060,443
Polyacrylate MW 4500 is supplied by BASF
Carboxymethyl cellulose is Finnfix^{®} V supplied by CP Kelco, Arnhem, Netherlands
CHEC is a cationically modified hydroxyethyl cellulose polymer.
Phosphonate chelants are, for example, diethylenetetraamine pentaacetic acid (DTPA) Hydroxyethane di phosphonate (HEDP)
Savinase^{®}, Natalase^{®}, Stainzyme^{®}, Lipex^{®}, CellucleanTM, Mannaway^{®} and Whitezyme^{®} are all products of Novozymes, Bagsvaerd, Denmark.
Purafect^{®}, Purafect Prime^{®} are products of Genencor International, Palo Alto, California, USA
Fluorescent Brightener 1 is Tinopal^{®} AMS, Fluorescent Brightener 2 is Tinopal^{®} CBS-X, Direct Violet 9 is Pergasol^{®} Violet BN-Z NOBS is sodium nonanoyloxybenzenesulfonate
TAED is tetraacetylethylenediamine
S-ACMC is carboxymethylcellulose conjugated with C.I. Reactive Blue 19product name AZO-CM-CELLULOSE
Soil release agent is Repel-o-tex^{®} PF
Acrylic Acid/Maleic Acid Copolymer is molecular weight 70,000 and acrylate:maleate ratio 70:30
EDDS is a sodium salt of ethylenediamine-N,N'-disuccinic acid, (S,S) isomer Suds suppressor agglomerate is supplied by Dow Corning, Midland, Michigan, USA
HSAS is mid-branched alkyl sulfate
Liquitint^{®} Violet CT polymeric hueing dye, supplied by Milliken, Spartanburg, South Carolina, USA
Polyethoxylated azo thiophene dye is Violet DD^{™} polymeric hueing dye, supplied by Milliken, Spartanburg, South Carolina, USA.
¹ Random graft copolymer is a polyvinyl acetate grafted polyethylene oxide copolymer having a polyethylene oxide backbone and multiple polyvinyl acetate side chains. The molecular weight of the polyethylene oxide backbone is about 6000 and the weight ratio of the polyethylene oxide to polyvinyl acetate is about 40 to 60 and no more than 1 grafting point per 50 ethylene oxide units.
² Polyethyleneimine (MW = 600) with 20 ethoxylate groups per -NH
³ Amphiphilic alkoxylated polymer is a polyethylenimine (MW 600), prepared from a polymer that is derivatised to contain 24 ethoxylate groups per -NH and 16 Propoxylate groups per -NH
⁴Amylase is shown as mgs of active enzyme per 100g of detergent.
⁵DNase as described herein (in all of these examples is shown as mgs of active enzyme per 100g of detergent). DNase may comprise minor amounts of super oxide dismutase impurity.
⁶Alginate lyase as described herein (in all examples shown as mgs of active enzyme per 100g of detergent)
⁷Hexosaminidase as described herein (in all examples shown as mgs of active enzyme per 100g of detergent)
⁸Pel-ase as described herein (in all examples shown as mgs of active enzyme per 100g of detergent)
⁹Psl-ase as described herein (in all examples shown as mgs of active enzyme per 100g of detergent)
¹⁰Endo-beta-1,3(4)-glucanase as described herein (in all examples shown as mgs of active enzyme per 100g of detergent).^{a} Proxel GXL, 20% aqueous dipropylene glycol solution of 1,2-benzisothiazolin-3-one, supplied by Lonza.
^{b} N,N-bis(hydroxyethyl)-N,N-dimethyl ammonium chloride fatty acid ester. The iodine value of the parent fatty acid of this material is between 18 and 22. The material as obtained from Evonik contains impurities in the form of free fatty acid, the monoester form of N,N-bis(hydroxyethyl)-N,N-dimethyl ammonium chloride fatty acid ester, and fatty acid esters of N,N-bis(hydroxyethyl)-N-methylamine.
^{c} MP10^{®}, supplied by Dow Corning, 8% activity
^{d} as described in US 8,765,659, expressed as 100% encapsulated perfume oil ^{e} Rheovis^{®} CDE, cationic polymeric thickener supplied by BASF
^{f} N,N-dimethyl octanamide and N,N-dimethyl decanamide in about a 55:45 weight ratio, tradename Steposol^{®} M-8-10 from the Stepan Company

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A detergent composition, preferably a laundry detergent composition, comprising (a) from 0.00005 to 5 wt% alginate lyase enzyme (active enzyme protein) and (b) 0.00005 wt% to 5 wt% enzyme (active enzyme protein) of a booster enzyme selected from one or more of (i) Pel-ase enzyme; (ii) Psl-ase enzyme; (iii) endo-β-1,3(4)-glucanase enzyme; and (iv) mixtures thereof.

2. A detergent composition according to claim 1 wherein the alginate lyase enzyme is of microbial origin, preferably bacterial or algal, most preferably bacterial.

3. A detergent composition according to claim 1 or claim 2 wherein the alginate lyase enzyme is obtainable from *Flavobacterium sp, Sphingomonas sp, Zobellia galactanivorans, preferably Flavobacterium sp.*

4. A detergent composition according to any preceding claim wherein the alginate lyase enzyme comprises: an alginate lyase selected from alginate lyase having at least 60%, or at least 70%, or at least 80%, or at least 90%, or at least 95%, or at least 96%, or at least 97%, or at least 98%, or at least 99%, or 100%, sequence identity to one or more of SEQ ID NO: 1; alginate lyase having at least 60%, or at least 70%, or at least 80%, or at least 90%, or at least 95%, or at least 96%, or at least 97%, or at least 98%, or at least 99%, or 100%, sequence identity to SEQ ID NO: 2; alginate lyase having at least 60%, or at least 70%, or at least 80%, or at least 90%, or at least 95%, or at least 96%, or at least 97%, or at least 98%, or at least 99%, or 100%, sequence identity to SEQ ID NO: 3; an alginate lyase having at least 60%, or at least 70%, or at least 80%, or at least 90%, or at least 95%, or at least 96%, or at least 97%, or at least 98%, or at least 99%, or 100%, sequence identity to SEQ ID NO: 4; alginate lyase having at least 60%, or at least 70%, or at least 80%, or at least 90%, or at least 95%, or at least 96%, or at least 97%, or at least 98%, or at least 99%, or 100%, sequence identity to SEQ ID NO: 5; alginate lyase having at least 60%, or at least 70%, or at least 80%, or at least 90%, or at least 95%, or at least 96%, or at least 97%, or at least 98%, or at least 99%, or 100%, sequence identity to SEQ ID NO: 6; alginate lyase having at least 60%, or at least 70%, or at least 80%, or at least 90%, or at least 95%, or at least 96%, or at least 97%, or at least 98%, or at least 99%, or 100%, sequence identity to SEQ ID NO: 7, and mixtures thereof, preferably wherein the alginate lyase enzyme comprises: an alginate lyase selected from alginate lyase having at least 60% sequence identity to one or more of SEQ ID NO: 1; SEQ ID NO: 5; SEQ ID NO: 6; and SEQ ID NO: 7, or mixtures thereof, most preferably wherein the alginate lyase enzyme comprises: an alginate lyase selected from alginate lyase having at least 60%, sequence identity to one or more of SEQ ID NO: 6; and SEQ ID NO: 7 and mixtures thereof.

5. A detergent composition according to any preceding claim additionally comprising surfactant, preferably wherein the surfactant is present in an amount such that the weight ratio of surfactant to active alginate lyase enzyme protein is at least 500:1, preferably at least 1000:1.

6. A laundry detergent composition according to any preceding claim wherein the alginate lyase enzyme provides activity towards poly(beta-D-mannuronate) (polyM activity) and activity towards poly(alpha-L-guluronate) (polyG activity).

7. A detergent composition according to any preceding claim additionally comprising an enzyme selected from nuclease enzyme, hexosaminidase enzyme, endo-β-1,3-glucanase enzyme, and mixtures thereof, the or each enzyme being present in an amount from 0.00005 to 5 wt% active enzyme protein.

8. A detergent composition according to any preceding claim wherein the composition additionally comprises nonionic surfactant, preferably in an amount from 1 to 30 wt% of the composition.

9. A detergent composition according to any preceding claim wherein the composition additionally comprises surfactant, said surfactant comprising an anionic and a nonionic surfactant, preferably in a weight ratio of anionic to nonionic surfactant of from 30:1 to 1:2, preferably from 20:1 to 2:3, or to 1:1.

10. A detergent composition according to any preceding claim wherein the booster enzyme comprises (i) Pel-ase enzyme and Psl-ase enzyme, and optionally endo-β-1,3(4)-glucanase enzyme; (ii) Pel-ase enzyme and endo-β-1,3(4)-glucanase enzyme, and optionally Psl-ase; or (iii) endo-β-1,3(4)-glucanase enzyme and Psl-ase enzyme, and optionally Pel-ase enzyme.

11. A detergent composition according to any preceding claim comprising additional enzyme, preferably selected from amylase, nuclease, hexosaminidase, mannanase, xanthan lyase, xanthanase, and mixtures thereof.

12. A method of treating a fabric, the method comprising contacting a fabric with an aqueous wash liquor comprising (a) an alginate lyase enzyme; (b) 0.00005 wt% to 5 wt% enzyme (active enzyme protein) of a booster enzyme selected from one or more of (i) Pel-ase enzyme; (ii) Psl-ase enzyme; (iii) endo-β-1,3(4)-glucanase enzyme; and (iv) mixtures thereof; and (iii) optionally a cleaning adjunct.

13. Use of a composition or method according to any of claims 1 to 12 for improving whiteness of a fabric; and/or for improved soil removal from a fabric; and/or for malodour removal from a fabric; and/or for cleaning collar and/or cuffs; and/or for anti-wrinkle benefits on a fabric; and/or for improved drying of a fabric.
